# EUROPEAN PATENT APPLICATION

(11) **EP 4 052 662 A1**
(43) Date of publication of application: **07.09.2022**
(21) Application number: 20887928.8
(22) Date of filing: 10.11.2020
(51) Int. Cl.: A61B 17/122

(54) **MULTIPLE PROCESSING DEVICE FOR ENDOSCOPE AND USE METHOD THEREOF**

(30) Priority: 14.11.2019 CN 201911111454; 18.02.2020 CN 202010099167
(71) Applicant: Hangzhou AGS MedTech Co., Ltd., Hangzhou, Zhejiang, 311106 (CN)
(72) Inventor: SHI, Bingchun, Hangzhou, Zhejiang 311106 (CN); SHI, Baiming, Hangzhou, Zhejiang 311106 (CN)
(74) Representative: Petraz, Gilberto Luigi
(86) International application number: PCT/CN2020/127767
(87) International publication number: WO 2021/093723

(57) **Abstract**

Provided is a multiple processing device (100) for endoscope, including at least two processing units (11), a delivery component and an operating component. The processing unit (11) includes a capsule (111) and a clamping arm (112), the proximal end of the capsule (111) is provided with a first limiting portion (40, 40a, 40b) that can be deformed or failed under the action of an external force. The distal end of a delivery pipe (21) and/or the proximal end of the capsule (111) is also provided with a second limiting portion (50, 50a, 50b, 50c, 50d) that can expand and contract along the radial direction of the delivery pipe (21). The multiple processing device (100) for endoscope enhances the combination between the capsule (111) and the delivery pipe (21), so as to facilitate the opening and closing operations of the clamping arm (112), and also prevents the processing unit (11) from interfering or jamming the inner wall of the delivery pipe (21) due to the expanded first limiting portion (40, 40a, 40b), which can ensure that the processing unit (11) moves smoothly in the delivery pipe (21), so that the processing unit (11) can be effectively repeatedly opened and closed before being released. Provided is also a use method of the multiple processing device (100) for endoscope.

## Description

### CROSS-REFERENCE TO RELATED APPLICATIONS

This application claims all benefits accruing from China Patent Application No. 201911111454.5, filed on November 14, 2019, titled "A MULTIPLE PROCESSING DEVICE FOR AN ENDOSCOPE AND METHODS OF USE", and No. 202010099167.3, filed on February 18, 2020, titled "A MULTIPLE PROCESSING DEVICE FOR AN ENDOSCOPE AND METHODS OF USE" in the China National Intellectual Property Administration, the content of which is hereby incorporated by reference.

### TECHNICAL FIELD

The present invention relates to a medical device, in particular to a multiple processing device for an endoscope and use thereof.

### BACKGROUND

As an endoscope was appeared more than 50 years ago, and has developed widely in the fields of disease diagnosis and even to disease treatment, the endoscope is so effective and reliable at treating diseases. Among them, flexible endoscopes have been widely used in digestion, gynecology, urology, respiratory tract and cardiovascular field because it does not require a surgical opening and has a characteristic of minimally invasive. At the same time, a technical requirement for the combination of miniaturization, maneuverability and high flexibility of a surgical instrument used in conjunction with the flexible endoscope has been proposed. Stomach and intestines of an organism often bleed, mucosal damage or even perforation due to various diseases, accidental damage or damage caused by the endoscopic treatment. Clinically, mechanical compression hemostatic method with a hemostatic clip can be used to stop bleeding. Hemostatic clips can grasp the tissue around the wound and temporarily hold the wound edges together to close the wound. The hemostatic clips are also used for wound suture. More than one hemostatic clip is sometimes required during surgery, and frequent instrument switching will reduce surgical efficiency and elevate surgical risk.

Since the diameter of the endoscopic channel is between 2.8 mm and 3.2 mm, and is able to bend more than 180° during the reverse mirror surgery. It is a problem that conventional multiple hemostatic clip needs to overcome that how to ensure the precise combination of free rotation, delivery, forward-reverse switching and repeated opening and closing functions of the minimally invasive multiple hemostatic clip under the above extreme bending state. The conventional multiple hemostatic clips are provided with a plurality of clip units inside a delivery tube. A rear clip unit may be jammed by the interference of a front clip unit. Multiple clip units inside the delivery tube are not able to pass smoothly through the lumen channel of the curved delivery tube in the extreme bending state due to being built into the delivery tube, and also are not able to achieve stable and repeatable opening and closing operation after the clip unit extends out of the delivery tube.

### SUMMARY

In accordance with a first aspect of the present application, the present invention provides a multiple processing device for endoscope. The multiple processing device for endoscope includes at least two processing units. Each of the at least two processing units includes a capsule and a clamping arm, the clamping arm is accommodated in the capsule and has an opened state and a closed state, the clamping arm is capable of switching between the opened state and the closed state, and a proximal end of the capsule is provided with a first limiting portion that is capable of deforming or out of operation under an action of an external force. A delivery component, wherein the delivery component includes a delivery pipe for accommodating the at least two processing units, a distal end of the delivery pipe is provided with a limiting stage protruding towards an axis of the delivery pipe, and a manipulation member penetrating through the delivery pipe, and the manipulation member is capable of driving a distal processing unit of the at least two processing units to switch from a combined state to a separated state, wherein in the combined state, the distal processing unit of the at least two processing units is combined with the distal end of the delivery pipe, and in the separated state, the distal processing unit is separated from the distal of the delivery pipe; and an operating component, wherein the operating component includes a handle portion combined with a proximal end of the delivery pipe, and a slidable portion sleeved on the handle portion and combined with a proximal end of the manipulation member, and the slidable portion is configured to drive the manipulation member to move. Wherein the distal end of the delivery pipe and/or the proximal end of the capsule are further provided with a second limiting portion abutting against with the limiting stage and being capable of contracting or expanding along the radial direction of a cross section of the delivery pipe.

In some embodiments, the second limiting portion is disposed at the distal end of the delivery pipe, when the distal processing unit of the at least two processing units is in the combined state, the second limiting portion is located at a proximal end of the limiting stage, the first limiting portion is provided with a thin portion closed to an axis of the capsule, and the thin portion is configured to abut against the second limiting portion and limit the capsule.

In some embodiments, when the distal processing unit of the at least two processing units is in the separated state, an outer diameter of the first limiting portion is smaller than a smaller one selected from a diameter of a first through hole defined by the limiting stage and an inner diameter of the second limiting portion.

In some embodiments, the first limiting portion is provided with an extending portion for accommodating the second limiting portion and an elastic member sleeved on a proximal end of the extending portion, and an inner surface of the elastic member abuts against an outer surface of the extending portion and is configured to converge the thin portion.

In some embodiments, the first limiting portion includes a first substrate and a first protrusion, the first substrate is accommodated in the proximal end of the capsule, and the first protrusion is connected to the first substrate and protrudes outwards, and the first protrusion protrudes out from a sidewall of the capsule and is limited near the limiting stage.

In some embodiments, an angle defined by the first substrate and the first protrusion is equal to or smaller than 30°, and an end surface of a proximal end of the first substrate is provided with a deforming portion, which is configured to make the first substrate deform or be out of operation under the external force.

In some embodiments, the deforming portion penetrates through the end surface of the proximal end of the first substrate.

In some embodiments, a distal end of the manipulation member expanding is defined as a head portion, a second through hole is defined at a center of the first substrate, and the head portion of the manipulation member is capable of penetrating through the second through hole, and a minimum inner diameter of the second through hole is smaller than a maximum outer diameter of the head portion of the manipulation member.

In some embodiments, each of the at least two processing unit further include a pedestal, which is fixed to the proximal end of the capsule, the pedestal includes an accommodation chamber configured to accommodate the first substrate, and a sidewall of the pedestal is provided with a third through hole configured for the first protrusion to extend outwards.

In some embodiments, when the distal processing unit of the at least two processing units is in the combined state, the first limiting portion is located in the limiting stage and clipped to the limiting stage, when the distal processing unit of the at least two processing units is in the separated state, the first limiting portion is deformed or out of operation under the action of the manipulation member and is unclipped from the limiting stage, a diameter of the first through hole defined by the limiting stage is greater than an outer diameter of a distal end of the first limiting portion, and smaller than a diameter of a proximal end of the second limiting portion.

In some embodiments, the inner diameter of the second limiting portion decreases from the proximal end of the delivery pipe to the distal end of the delivery pipe.

In some embodiments, the second limiting portion is disposed at the distal end of the delivery pipe, and accommodated in the proximal end of the limiting stage, when the second limiting portion of the at least two processing units is in a state without radial force, an inner diameter of a distal end of the second limiting portion is smaller than an outer diameter of the at least two processing units, and an inner diameter of a proximal end of the second limiting portion is larger than the outer diameter of the at least two processing units.

In some embodiments, when the distal processing unit of the at least two processing units is in the combined state, a distal end of the second limiting portion abuts against the thin portion, and the proximal end of the limiting stage abuts against a distal end of the first limiting portion, the manipulation member is configured to make the clamping arm move while switching between the opened state and the closed state. The distal processing unit of the at least two processing units is in the separated state, a proximal end of the slidable portion is capable of moving to release a combination between the manipulation member, the at least two processing units and the first limiting portion, so that a combination between the first limiting portion and the limiting stage is out of operation.

In some embodiments, the second limiting portion is disposed at the distal end of the delivery pipe, and the second limiting portion includes a plurality of teeth portions which are disposed at intervals along a circumference of the delivery pipe and gathered towards the distal end of the delivery pipe, distal ends of the plurality of teeth portions are configured to abut against the thin portion, and proximal ends of the plurality of teeth portions are configured to abut against the expanded first limiting portion.

In some embodiments, the second limiting portion includes a second substrate and a second protrusion, the second substrate is accommodated in the capsule, the second protrusion is connected to the second substrate and extended outwards, and the second protrusion is extended towards a sidewall of the capsule and limited to the limiting stage.

In some embodiments, the number of the second protrusion is multiple, and a plurality of second protrusions are disposed along at intervals a circumference of the second substrate.

In some embodiments, a sidewall of the second limiting portion is provided with a first opening which is capable of providing a deformation allowance, and the first opening penetrates through the sidewall of the second limiting portion; and/or the sidewall of the second limiting portion is further provided with a second opening extending from an end surface of a proximal end of the sidewall of the second limiting portion to the sidewall of the second limiting portion, the number of the second opening is multiple, and a plurality of the second openings are disposed at intervals along a circumference of the second limiting portion.

In some embodiments, a distal end of the manipulation member includes a head portion, a concave portion and a shoulder portion; and a proximal end of the clamping arm is provided with a clamping member through which the concave portion penetrates, and the clamping member abuts against a proximal end of the head portion of the manipulation member and a distal end of the shoulder, respectively, and the clamping member is out of operation under a predetermined force.

In some embodiments, the distal end of the shoulder inclines inwards the axis of the delivery pipe, so as to ensure that the manipulation member is clamped to the proximal end of the clamping member, so as to drive the at least two processing units to rotate.

In some embodiments, the clamping member includes a pair of pins penetrating through the proximal end of the clamping arm, each of the pair of pins includes a first end and a second end, and the second end of each of the pair of pins is a movable end, the first end of each of the pair of pins is U-shaped via an integrally forming process, or the first end is provided with a limiting strip, which is configured to limit the pair of pins and fix the first end of each of the pair of pins to the clamping arm.

In some embodiments, the clamping member includes a folded portion, which is defined by an arm surface of the clamping arm bending inwards, and the folded portion and the clamping arm are enclosed to define two end surfaces configured to abut against the concave portion.

In some embodiments, each of the at least two processing units further includes a limiting member disposed between two clamping arms, when the clamping arm is in the closed state, the manipulation member penetrates through the limiting member towards a direction of the head portion abutting against the clamping member under restriction of the limiting member.

In some embodiments, a part of a sidewall of the proximal end of the clamping arm protruding outwards is defined as a locking portion, a part of a sidewall of the capsule bending inwards is defined as a clamping hole portion, and the clamping hole portion matches with the locking portion and is capable of limiting the clamping arm from moving towards a distal end of the capsule.

In some embodiments, the delivery pipe includes a first pipe near the distal end of the delivery pipe and a second end near the proximal end of the delivery pipe, and the first pipe is fixed to the second pipe. The first pipe is made by winding ribbon-shaped wires and the second pipe is made by winding round-shaped wires, and the first pipe is configured to accommodate the at least two processing units.

In accordance with a second aspect of the present application, the present invention provides a multiple processing device for endoscope. The multiple processing device for endoscope includes: a processing component, wherein the processing component includes at least two processing units, and a proximal end of each of the at least two processing units is provided with a first limiting portion that is capable of deforming or out of operation under an action of an external force; a delivery component, wherein the delivery component includes a delivery pipe accommodated in the processing component, a pushing pipe penetrating through the delivery pipe and a manipulation member penetrating through the pushing pipe, wherein a distal end of the pushing pipe abuts against a proximal processing unit of the at least two processing units; and an operating component. The operating component includes: a handle portion combined with a proximal end of the delivery pipe; a slidable portion combined with a proximal end of the manipulation member, which is configured to control a distal processing unit of the of at least two processing units to open and close; and a pushing component disposed on the handle portion, wherein the pushing component includes a fixing seat fixed to a proximal end of the delivery pipe. The handle portion is provided with a sliding groove for the fixing seat to slide, and the handle portion is provided with a marking portion configured for matching with the fixing seat and showing the number of the at least two processing units.

In some embodiments, the pushing component further includes a locking member and a resetting member, the locking member is connected to the fixing seat, the resetting member is disposed between the fixing seat and the locking member and abuts against the locking member, the locking member is provided with a first locking portion, the handle portion is provided with a second locking portion, and the first locking portion is capable of meshing with or separating from the second locking portion, so as to lock or unlock the locking member with the handle portion.

In some embodiments, the handle portion is provided with a plurality of marking portions, which are disposed in equidistance along a moving direction of the fixing seat; and the plurality of marking portions are figure notations, which are configured to show the number of remaining processing units.

In some embodiments, the fixing seat is provided with a guiding column extending towards the locking member, the locking member is provided with a guiding groove through which the guiding column inserts, and the locking member is capable of moving along an axis of the guiding column relative to the locking member.

In some embodiments, the locking member is further provided with an extension portion stretching out from a sidewall of the handle portion in a locked state, and the sidewall of the handle portion is provided with a sliding groove, wherein the extension portion is capable of stretching out from the sliding groove and sliding along the sliding groove.

In some embodiments, the pushing component further includes a crimper, which is sleeved on the pushing pipe and fixed to the fixing seat.

In some embodiments, the number of the crimper is two, and the two crimpers are fixed to two ends of the fixing seat, respectively.

In accordance with a third aspect of the present application, the present invention further provides a method of use of a multiple processing device for endoscope. The multiple processing device for endoscope includes a processing component, a delivery component and a pushing component, wherein the processing component includes at least two processing units. The pushing component includes a delivery pipe configured for accommodating the processing component, a pushing pipe penetrating through the delivery pipe, and a manipulation member penetrating through the pushing pipe, wherein a distal end of the pushing pipe abuts against a most proximal processing unit, and the pushing component is fixed to a proximal end of the pushing pipe. The method includes the following steps: releasing a distal processing unit in the delivery pipe, and driving the manipulation member to move towards a proximal end of the delivery pipe, until a first user feedback, which shows a more proximal processing unit is combined with a distal end of the manipulation member, is obtained; driving the pushing component to move towards a distal end of the delivery pipe, and driving the first processing unit to move towards a distal end of the delivery pipe via the pushing pipe fixed to the pushing component, until a second user feedback, which shows the first processing unit is combined with the distal end of the delivery pipe, is obtained; after the second user feedback is obtained, driving the distal processing component with the operating component to freely open or closed to obtain an expected state.

In some embodiments, the pushing component includes a fixing seat, a locking member and a resetting member, wherein the resetting member is disposed between the fixing seat and the locking member and abuts against the fixing seat and the locking member, wherein the locking member is provided with a first locking portion matching with a second locking portion disposed on the handle portion. The step of driving the pushing component to move towards a distal end of the delivery pipe, and driving the proximal processing unit to move towards a distal end of the delivery pipe via the pushing pipe fixed to the pushing component, until a second user feedback, which shows the proximal processing unit is combined with the distal end of the delivery pipe, is obtained further includes: when the pushing pipe is moved, pressing the locking member to make the first locking portion and the second locking portion being unlocked; and after the second user feedback is obtained, releasing the locking member to make the first locking portion and the second locking portion being locked.

Compared with conventional technique, the present invention has the following advantages. By providing a first limiting portion that is capable of deforming or out of being operation under an action of an external force at a proximal end of the capsule, providing a limiting stage clipped to the first limiting portion at a distal end of the delivery pipe, and the first limiting portion is clipped to the limiting stage, the clamping arm can switch between a opened state and a closed state. The distal end of the delivery pipe and/or the proximal end of the capsule are further provided with a second limiting portion. The second limiting portion is located on an inner side or an outer side of the limiting stage, and is capable of contracting or expanding along the axis of the delivery pipe to ensure that the distal end of the capsule passes through the limiting stage. On the one hand, enhancing the clip between the first limiting portion and the delivery pipe, and on the other hand, limiting the movement of the capsule relative to the proximal end of the delivery pipe. The multiple processing device for endoscope ensures both that the at least two processing units move smoothly inside the delivery pipe and that the first limiting portion does not interfere with or jam the inner wall of the delivery pipe, and that the effective repeated opening and closing of the at least two processing units before it is released is achieved by acting with the first limit section.

Since the manipulation member is able to effectively loaded with each of the at least two processing units, especially with the a first processing unit, and repeatedly opened and closed, the release process of each of the at least two processing units can be ensured to be stable and reliable. The multiple processing device for endoscope is able to be inserted once to release multiple processing units for clamping the lesion, which greatly reduces the operating time and discomfort during a surgery.

In addition, the present invention further provides a multiple processing device for endoscope. The pushing component disposed on the handle portion is able to act on the pushing pipe and drive the pushing pipe to move synchronously so that the processing unit located on the proximal end of the delivery pipe can move toward the distal end and the manipulation member can effectively load and operate the processing unit located outside of its loading stroke. Thereby the number of the processing units in the delivery pipe can increase and ensure that each of the processing unit can be effectively loaded.

The present invention further provides a method for use a multiple processing device for endoscope. The multiple processing device for endoscope can perform an operation of repeatable opening and closing

### BRIEF DESCRIPTION OF THE DRAWINGS

In order to better describe and explain the embodiments and/or examples of those inventions disclosed herein, one or more drawings may be referred to. The additional details or examples used to describe the drawings should not be considered as limiting the scope of any of the disclosed inventions, the currently described embodiments and/or examples, and the best mode of these inventions currently understood.
FIG. 1 is a structural schematic diagram of a multiple processing device for an endoscope from a first view angle in an embodiment.
FIG. 2 is a section view of the multiple processing device for the endoscope in FIG. 1.
FIG. 3 is a structural schematic diagram of the multiple processing device for the endoscope in FIG. 1 from a second view angle.
FIG. 4 is a section view of the multiple processing device for the endoscope in FIG. 3.
FIG. 5 is an enlarged view of portion A in FIG. 4 in an embodiment.
FIG. 6 is an enlarged view of portion A in FIG. 4 in another embodiment.
FIG. 7 is a structural schematic diagram of a mounting portion in an embodiment.
FIG. 8 is a structural schematic diagram of a multiple processing device for an endoscope, wherein a part of the structures are hidden.
FIG. 9(a) is a stereo structural schematic diagram of a first limiting portion in an embodiment.
FIG. 9(b) is a schematic diagram of a first limiting portion in one of the use states in an embodiment.
FIG. 9(c) is a schematic diagram of a first limiting portion in one of the use states in an embodiment.
FIG. 10 is a structural schematic diagram of a manipulation member in an embodiment.
FIG. 11 is a structural schematic diagram of a second limiting portion in an embodiment.
FIG. 12 is a structural schematic diagram of a second limiting portion in an embodiment.
FIG. 13 is a structural schematic diagram of a second limiting portion in an embodiment.
FIG. 14 is an assembly diagram of a processing unit, a first limiting portion and a second limiting portion in an embodiment.
FIG. 15 is an assembly diagram of a capsule and a first limiting portion in an embodiment.
FIG. 16 is a stereo structural schematic diagram of a pedestal in an embodiment.
FIG. 17 is a structural schematic diagram of a second limiting portion in an embodiment.
FIG. 18 is an assembly diagram of a processing unit, a first limiting portion and a second limiting portion in an embodiment.
FIG. 19 is a schematic diagram of FIG. 18 from another view angle.
FIG. 20 is a section view of a multiple processing device for an endoscope in an embodiment.
FIG. 21 is an enlarged view of portion B in FIG. 20.
FIG. 22 is an assembly diagram of a capsule and a first limiting portion in an embodiment.
FIG. 23 is an assembly diagram of a capsule and a first limiting portion in an embodiment.
FIG. 24 is a structural schematic diagram of a limiting member in an embodiment.
FIG. 25 is a structural schematic diagram of a limiting member in an embodiment.
FIG. 26 is a structural schematic diagram of a clamping arm in an embodiment.
FIG. 27 is a structural schematic diagram of a clamping arm in an embodiment from another view angle.
FIG. 28 is an assembly diagram of a clamping member, a processing unit and a manipulation member in an embodiment.
FIG. 29 is a structural schematic diagram of a clamping member in an embodiment.
FIG. 30 is an assembly diagram of a clamping member, a processing unit and a manipulation member in an embodiment.
FIG.31 is a structural schematic diagram of a clamping arm in an embodiment.
FIG.32 is a structural schematic diagram of a clamping arm in a free state in an embodiment.
FIG.33 is a structural schematic diagram of a clamping arm in a self-locked state in an embodiment.
FIG.34 is a structural schematic diagram of a clamping arm in a free state in an embodiment.
FIG.35 is a structural schematic diagram of a processing unit in a self-locked state in an embodiment.
FIG. 36 is a structural schematic diagram of a multiple processing device for an endoscope in an embodiment.
FIG. 37 is a structural schematic diagram of the multiple processing device for the endoscope from a first view angle in FIG. 36.
FIG. 38 is a section view of FIG. 37.
FIG. 39 is a structural schematic diagram of the multiple processing device for the endoscope from a second view angle in FIG. 36.
FIG. 40 is a section view of FIG. 39.
FIG. 41 is a section view of a multiple processing device for an endoscope in a use state in an embodiment.
FIG. 42 is an enlarged view of portion C in FIG. 41.
FIG. 43 is an enlarged view of portion D in FIG. 41.
FIG. 44 is a section view of portion C in another use state.
FIG. 45 is an exploded view of a pushing member in an embodiment.
FIG. 46 is a structural schematic diagram of a fixing seat in an embodiment.
FIG. 47 is a structural schematic diagram of a locking member in an embodiment.
FIG. 48 is a structural schematic diagram of a handle portion in an embodiment.
FIG. 49 is a schematic diagram of a multiple processing device for an endoscope in a use state in an embodiment.
FIG. 50 is a schematic diagram of a multiple processing device for an endoscope in a use state in an embodiment.
FIG. 51 is a schematic diagram of a multiple processing device for an endoscope in a use state in an embodiment.
FIG. 52 is an enlarged view of portion E in FIG. 51.
FIG. 53 is a schematic diagram of a multiple processing device for an endoscope in a use state in an embodiment.
FIG. 54 is a schematic diagram of a multiple processing device for an endoscope in a use state in an embodiment.
FIG. 55 is a schematic diagram of a multiple processing device for an endoscope in a use state in an embodiment.
FIG. 56 is a schematic diagram of a multiple processing device for an endoscope in a use state in another embodiment.
FIG. 57 is a schematic diagram of a multiple processing device for an endoscope in a use state in another embodiment.
FIG. 58 is a schematic diagram of a multiple processing device for an endoscope in a use state in another embodiment.
FIG. 59 is a schematic diagram of a multiple processing device for an endoscope in a use state in another embodiment.
FIG. 60 is a schematic diagram of a multiple processing device for an endoscope in a use state in another embodiment.
FIG. 61 is a schematic diagram of a multiple processing device for an endoscope in a use state in another embodiment.

100 represents a multiple processing device for an endoscope; 10 represents a processing component; 11 represents a processing unit; 111 represents a capsule; 1111 represents a clamping hole portion; 1112 represents an opening hole; 112 represents a clamping arm; 1121 represents a locking portion; 1122 represents a locking seat; 113 represents a pedestal; 1131 represents a third through hole; 114 represents a limiting member; 12 represents a clamping member; 121 represents a pin; 1211 represents a limiting strip; 122 represents a folded portion; 13 represents a separating member; 20 represents a delivery component; 21 represents a delivery pipe; 211 represents a first pipe; 212 represents a second pipe; 22 represents a manipulation member; 221 represents a head portion; 222 represents a concave portion; 223 presents a shoulder portion; 23 represents a mounting portion; 230 represents a first through hole; 231 represents a base; 232 represents a limiting stage; 233 represents a teeth portion; 234 represents a fourth through hole; 235 represents a flange; 25 represents a pushing pipe; 30 represents an operating component; 31 represents a handle portion; 311 represents a second locking portion; 312 represents a sliding groove; 313 represents a marking portion; slidable portion-32 represents a slidable portion; represents a connecting rod; 322 represents a slidable ring; 323 represents a thumb ring; 40,40a and 40b represent a first limiting portion; 400 represents a cavity; 41a represents an extending portion; 42a represents a clipping portion; 42a1 represents clipping protrusion; 43a represents a thin portion; 44a represents an elastic member; 41b represents a first substrate; 41b0 represents a second through hole; 41b1 represents a deforming portion ; 42b represents a first protrusion; 50, 50a, 50b, 50c and 50d represent a second limiting portion; 51d represents a second substrate; 52d represents a second protrusion; 53 represents a first opening; 54 represents a second opening; 60 represents a pushing component; 61 represents a fixing seat; 611 represents a guiding column; 62 represents a locking member; 621 represents a first locking portion; 622 represents a guiding groove; 623 represents an extension portion; 63 represents a resetting member; and 64 represents a crimper.

### DETAILED DESCRIPTION

The technical solutions in the embodiments of the present invention are clearly and completely described in the following with reference to the accompanying drawings in the embodiments of the present invention. It is obvious that the described embodiments are only a part of the embodiments, but not all of the embodiments. All other embodiments obtained by those skilled in the art based on the embodiments of the present invention without departing from the inventive scope are the scope of the present invention.

It should be noted that when an element is referred to as being "disposed" on another element, it may be directly disposed on the other element or a further element may be presented between them. When an element is considered to be "connected" to another element, it may be directly connected to the other element or connected to the other element through a further element. When an element is referred to as being "fixed" to another element, it may be directly attached to the other element or a further element may be presented between them.

It should be understood that in the description of this application, the orientation or positional relationship indicated by the orientation words involved is generally based on the orientation or positional relationship shown in the accompanying drawings and is intended only to facilitate and simplify the description of this application. In the absence of a statement to the contrary, these orientation words do not indicate and imply that the device or element referred to must have a particular orientation or be constructed and operated in a particular orientation, and therefore cannot be construed as limiting the scope of this application. The orientation words "inside and outside" refer to the inside and outside relative to the contours of the parts themselves.

Unless otherwise defined, all technical and scientific terms used herein have the same meaning as a skilled person in the art would understand. The terminology used in the description of the present invention is for the purpose of describing particular embodiments and is not intended to limit the invention. The term "or/and" as used herein includes any and all combinations of one or more of the associated listed items.

It should be noted that "proximal end" and "proximal side" are used herein to refer to the end or side of an element in a surgical procedure that is closer to an operator and is the opposite end or side of the terms "distal end" and "distal side". "Distal end" and "distal side" are used herein to refer to the end or side of an element in the surgical procedure that is farther from an operator and is the opposite end or side of the terms "proximal end" and "proximal side". In the present invention, the terms "first", "second", etc. are only used for distinguishing description, only for distinguishing one entity or operation from another entity or operation, and does not necessarily require or imply any such actual relationship or order between these entities or operations.

In conventional art, a multiple processing device for an endoscope can successively release a plurality of processing units into a living body, and stitch a wound by grabbing tissues around the wound and clamping an edge of the wound. Before the processing unit is released, the processing unit is moved back and forth and rotated to adjust clamping state of lesion. When the processing unit is moved back and forth, a distal end of the processing unit is switched between an opened state and a closed state, so as to repeatedly open and close the processing unit. When the processing unit is rotated, a clamping angle of the processing unit can be adjusted. Thus, accuracy of clamping operation is improved, and the processing unit can better aim at the lesion and effectively clamp the lesion.

The processing unit enters the body and is delivered to the lesion through the flexible endoscope of 1 meter to 2 meters. The flexible endoscope will be bent at a large angle in the organism, which is easily cause a situation where the processing unit of a conventional multiple processing device for endoscope will not be able to go through a bend, or will not be able to transmit torque to a distal end of the processing unit.

In addition, the conventional multiple processing device for endoscope is able to be inserted once to release multiple processing units for clamping the lesion. But after the release of a second processing unit, it is difficult for a first processing unit to load effectively with the manipulation member and achieve repeated opening, resulting in a failure or limitation of a multiple function. Therefore, it is important to effectively load each of the processing unit to ensure a clamping operation of the processing unit as well as stable release.

At present, the processing unit can be repeatedly opened and closed before being released by providing a limiting stage at the proximal end of the processing unit that is able to be elastically deformed. Specifically, the manipulation member abuts against the limiting stage and can make the limiting stage expand along a radial direction of a cross section of the limiting stage. When the manipulation member is disengaged from the limiting stage, the limiting stage can be radially contracted. The limiting stage fits the distal end of the delivery pipe by elastic deformation, so that when the processing unit repeatedly opens and closes to clamp the lesion, the limiting stage at the proximal end of the processing unit can be restrained at the distal end of the delivery pipe. However, if the radial deformation of the limiting stage of the conventional multiple processing device is large, the elastically deformed limiting stage will interfere with the inner wall of the delivery pipe, which affects the smooth movement of the processing unit in the delivery pipe, and is not conducive to the release of the processing unit into the organism from the delivery pipe. If the radial deformation of the limiting stage of the conventional multiple processing device is small, it is easy to detach from the distal end of the delivery pipe, which is not conducive to repeated opening and closing operations.

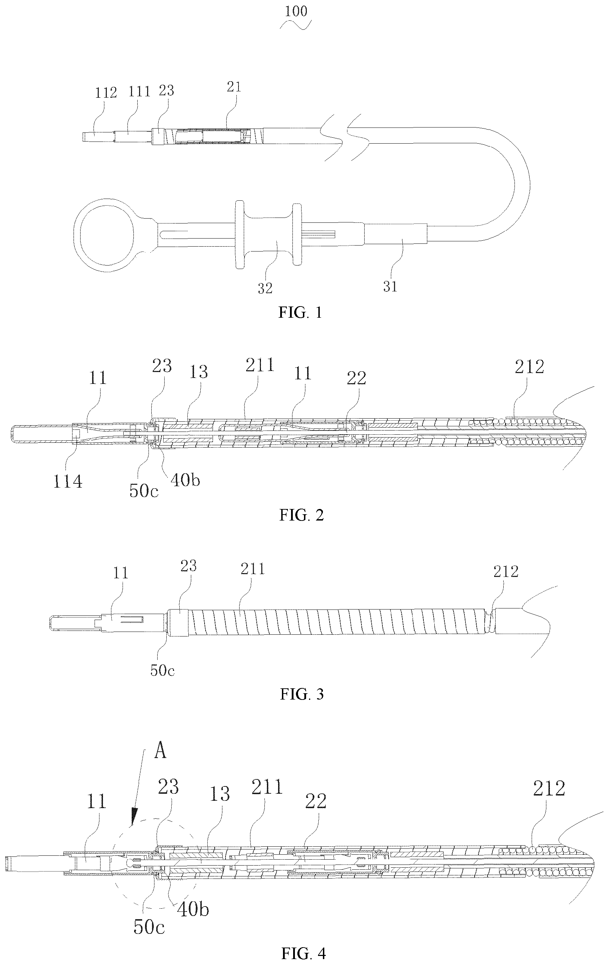

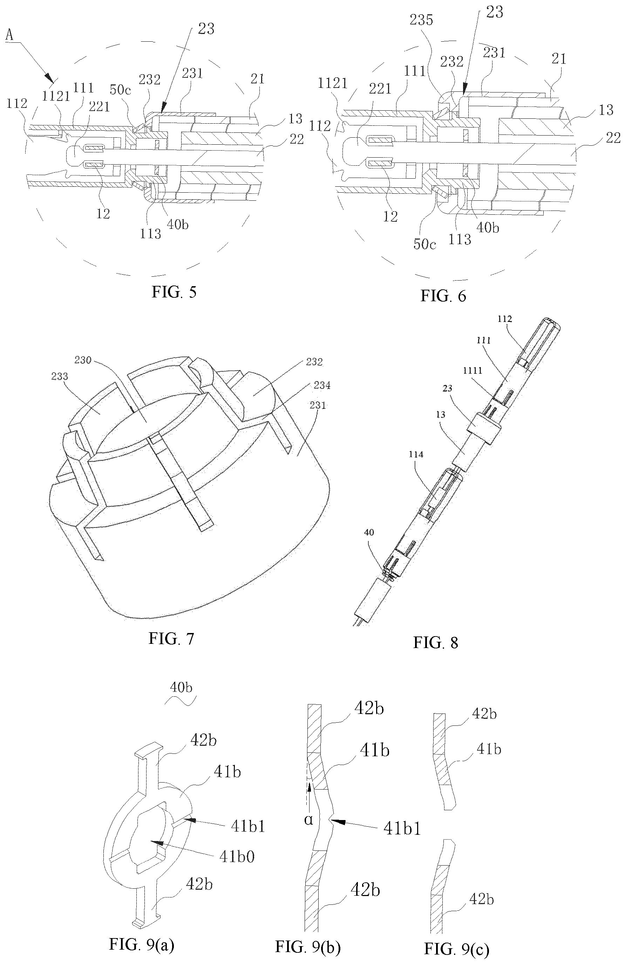

Referring to FIG. 1 to FIG. 5, the present invention provides a multiple processing device for an endoscope 100, which not only does not interfere an inner wall of a delivery pipe 21, but also can be repeatedly opened and closed. FIG. 1 is a structural schematic diagram of a multiple processing device for an endoscope 100 from a first view angle in an embodiment. FIG. 2 is a section view of the multiple processing device for the endoscope 100 in FIG. 1. FIG. 3 is a structural schematic diagram of the multiple processing device for the endoscope 100 in FIG. 1 from a second view angle. FIG. 4 is a section view of the multiple processing device for the endoscope 100 in FIG. 3. FIG. 5 is an enlarged view of FIG. 4.

The multiple processing device for an endoscope 100 includes a delivery component, an operation component, a processing unit 11, a first limiting portion 40b and a second limiting portion 50c.

In some embodiments, the delivery component includes a delivery pipe 21, a manipulation member 22 and a mounting portion 23.

The delivery pipe 21 includes at least one processing units 11, so that a plurality of processing units 11 is successively released into a living body at once when it is inserted into the living body. In some embodiments, the number of the at least one processing units 11 is in a range of 2 to 10.

In some embodiments, the delivery pipe 21 is an elastic flexible pipe, but is not limited to the elastic flexible pipe. An annular tube can be sleeved on the delivery pipe, and the annular tube can be made of a flexible material. In some embodiments, the delivery pipe 21 includes a first pipe 211 and a second end 212 combined with a proximal end of the second pipe 212. The first pipe 211 is made by winding ribbon-shaped wires and the second pipe 212 is made by winding round-shaped wires. For example, a first pipe 211 is made of a metal wire having a cross section in quadrangle-shaped. Compared with a second pipe 212 made of a metal wire having a cross section in a round shape, on premise that the an outer diameter of first pipe 211 and an outer diameter of the second pipe 232 are the same, a thickness of the sidewall of the first pipe 211 is smaller, and an inner diameter of the first pipe 211 is greater, which facilitates the at least one processing units 11 to move in the first pipe 211. Meanwhile, the first pipe 211 has a relatively great driving force which facilitates the clamping operation of a distal end of the delivery pipe 21 in the living body.

In some embodiments, the first pipe 211 and the second pipe 212 are fixed by welding. It should be understood that in some embodiments, the first pipe 211 and the second pipe 212 can be connected by other methods such as screwing, riveting, gluing, clipping and the like. By designing a delivery pipe 211 having a first pipe and a second pipe, the delivery pipe 211 not only has necessary driving force and accommodation space, but also has a low production cost.

The manipulation member 22 can penetrate through the delivery pipe 21, and can drive the at least one processing units 11 to switch from a combined state to a separated state. When the at least one processing units 11 are in the combined state, the distal processing unit 11 is combined with the distal end of the delivery pipe 21; and when the at least one processing units 11 are in the separated state, the distal processing unit 11 is separated from the distal end of the delivery pipe 21. The manipulation member 22 is a rope or pipe made by winding a plurality of metal wires having good torque transmission performance, or an independent metal wire or pipe. The manipulation member has a diameter in a range of 0.2 mm to 1.0 mm, and can achieve a 1:1 torque transmission. The manipulation member 22 can move back and forth or rotate in the delivery pipe 21, and can drive a processing unit 11 combined thereon to simultaneously move back and forth or rotate.

The mounting portion 23 is fixed on the distal end of the delivery pipe 21, and includes a limiting stage 232 extending towards an axis of the delivery pipe 21. Before being released, each of the plurality of processing units 11 accommodated in the delivery pipe 21 will penetrate through the limiting stage 232, and clipping with the limiting stage 232, so that the manipulation member 22 can make the clamping arm 112 open and close and rotate. After the processing unit 11 is separated from the limiting stage, the processing unit 11 is released from the delivery pipe 21 and enters a living body.

The operating component includes a handle portion 31 and a slidable portion 32 sleeved on the handle portion 31. The handle portion 31 is combined with a proximal end of the delivery pipe 21, and configured to drive the delivery pipe 21 to move simultaneously. The slidable portion 32 is connected to a proximal end of the manipulation member 22, and drives the manipulation member 22 and the processing unit 11 to move simultaneously. Therefore, a user can adjust an angle and a distance between the processing unit 11 and a lesion by moving the handle portion 31 and the slidable portion according to a condition of the lesion, so as to better aim at and clamp the lesion.

The processing unit includes a capsule 111 and the clamping arm 112, and a proximal end of the clamping arm is accommodated in the capsule 111. When the processing unit 11 is in the combined state, the proximal end of the clamping arm 112 is connected to the manipulation member 22, and can move forth and back relative to an axis of the capsule 111 under action of the manipulation member 22. At the same time, a distal end of the clamping arm 112 can be switched between the opened state and the closed state. After the clamping operation is completed, the processing unit 11 is in the closed state and is fixed to the capsule 111.

In order to make a distal end of the manipulation member 22 effectively combine with a first processing unit 11 closer to an operator after a second processing unit 11 farther from the operator is released, and make each of the at least one processing units 11 be able to be repeatedly opened and closed to more effectively clamp the lesion, the multiple processing device for the endoscope 100 is provided with a first limiting portion 40b and a second limiting portion 50c.

The first limiting portion 40b is disposed at a proximal end of the capsule 111, and is capable of deforming or out of operation under an action of an external force, thereby the first limiting portion 40b is combined with or separated from the limiting stage 232. The first limiting portion 40 is disposed at a proximal side of the limiting stage 232 and is clipped to the limiting stage 232, and can prevent the processing unit 11 from moving towards a distal end of the delivery pipe 21. When the processing unit 11 is in the combined state, the manipulation member 22 can make the clamping arm 112 move while switching between the opened state and the closed state. When the processing unit 11 is in the separated state, the first limiting portion 40 can be deformed and separated from the limiting portion 232.

The first limiting portion 40b can be defined with a relatively small outer diameter, so as to effectively clip with the limiting stage 232, while the processing unit 11 will not interference with an inner wall of the delivery pipe 21 or be stuck with the inner wall of the delivery pipe 21, thereby ensuring smoothly moving of the processing unit 11 in the delivery pipe 21. The first limiting portion 40b and the capsule 111 can be integrally formed or separately formed.

In the present embodiment, the second limiting portion 50c is sleeved on the proximal end of the capsule 111, and penetrated through and interference fit with the limiting stage 232 together with the capsule 111, and the first limiting portion 40b is kept at the distal end of the delivery pipe 21. When the processing unit 11 is in the combined state, a second limiting portion 50 is disposed at and abuts against a distal end of the limiting stage 232, so as to prevent the capsule 111 from moving towards the proximal end of the delivery pipe 21. The first limiting portion is disposed in the limiting portion 232 and clipped to the limiting stage 232, and the manipulation member 22 can make the clamping arm 112 move while switching between the opened state and the closed state. When the processing unit 11 is in the separated state, the first limiting portion is deformed and separated from the limiting stage 232 under action of the manipulation member 22. Therefore, when the first limiting portion 40 is expanded, the first limiting portion 40 can be better clipped to the proximal end of the limiting stage 232, so as to prevent unduly great or unduly small radial deformation of the first limiting portion 40.

It should be noted that in some embodiments, the second limiting portion 50 can be disposed at a distal end of the delivery pipe 21, and located at a proximal end of the limiting stage 232 when the processing unit 11 is in the combined state. In some embodiments, the number of the second limiting portion 50 can be two, and the two limiting portions 50 can be disposed at a distal end of the delivery pipe 21 and a proximal end of the capsule 111 at the same time.

That is, the second limiting portion 50 can be disposed at the distal end of the delivery pipe 21 and disposed in the limiting stage 232 when the processing unit 11 is in the combined state. In some embodiments, the second limiting portion 50 can be disposed at the proximal end of the capsule 111 and disposed outside the limiting stage 232 when the processing unit 11 is in the combined state. In some embodiments, the second limiting portion 50 can be disposed at the distal end of the delivery pipe 21 and the proximal end of the capsule 111 at the same time, thereby playing a twofold limiting role. Specifically, when the processing unit 11 is in the combined state, the second limiting portion 50 can expand or contract along a radial direction of the delivery pipe 21, and abut against the limiting stage 232, so that the capsule 111 can penetrate through the second limiting portion 50, and an expanded first limiting portion 40 is limited to the proximal end of the delivery pipe 21 so as to open and close the processing unit 11.

Furthermore, a diameter of a through hole defined by the limiting stage 232 is greater than an outer diameter of a distal end of the first limiting portion 40, and smaller than an outer diameter of a proximal end of the second limiting portion 50.

When the multiple processing device of the endoscope 100 is used, after the clamping operation, the user can draw the slidable ring 322 towards the proximal end of the delivery pipe 21, so that the clamping arm 112 is closed and locked in the capsule 111, and then continuously apply a driving force on the proximal end of the delivery pipe 21. When the applied driving force is larger than a preset driving force, the distal end of the manipulation member 22 is disconnected with the clamping arm 112, and the first limiting portion 40b is elastically contracted or deformed and separated from the limiting stage 232, thereby releasing a processing unit 11. The process can be repeated to release the at least one processing units 11 accommodated in the delivery pipe 21.

Referring to FIG. 5, the mounting portion 23 is fixed to the distal end of the delivery pipe 21. The mounting portion 23 and the delivery pipe 21 are integrally formed, or independently formed and fixed by methods such as screwing, welding riveting, gluing, and the like.

The mounting portion 23 includes a base 231 and a limiting stage 232defined by extending a distal end of the base 231 towards an axis of base 231. The base 231 is sleeved on the distal end of the delivery pipe 21, and an inner chamber is defined in the base 231 (not shown in the figures). The limiting stage 232 is provided a first though hole through which the capsule 111 and the second limiting portion 50c penetrate through, and the first through hole is configured to limit the first limiting portion.

Referring to FIG. 6, FIG. 6 is an enlarged view of portion A in FIG. 4 in another embodiment. The structures in FIG. 5 and FIG. 6 are basically the same. The differences between FIG. 5 and FIG. 6 are: the mounting portion in FIG. 6 further includes a flange 235. The flange 235 extends from the distal end of the limiting stage 232 towards the distal end of the mounting portion 23. An inner wall of the flange 235 and the distal end of the limiting stage 232 define a stepped face (not shown in the figures), which limits unduly expansion of the proximal end of the second limiting portion 50c.

In some embodiments, when the processing unit 11 is in the combined state, the capsule 111 and a second limiting portion 50c penetrate from through the frits through hole, and the second limiting portion 50c abuts against the end surface of the distal end of the limiting stage 232. Since the proximal end of the second limiting portion 50c expands relative to the distal end and has a larger outer diameter, the proximal end of the second limiting portion 50c and the outer surface of the capsule 111 does not contact with each other, when the second limiting portion 50c is driven by a driving force along a proximal end of the delivery pipe 21, the second limiting portion 50c may shake, and the structure is not stable enough.

By designing the flange 235, the proximal end of the second limiting portion 50c is accommodated in the distal end of the mounting portion 23, and the inner side of the flange 235 can limit the proximal end of the second limiting portion 50c, so as to prevent unduly shaking and make the structure stable.

Referring to FIG. 7 and FIG. 8, FIG. 7 is a structural schematic diagram of a mounting portion 23 in an embodiment; and FIG. 8 is a structural schematic diagram of a multiple processing device for an endoscope, wherein a part of the structures are hidden. The mounting portion 23 further includes a teeth portion 233, which is defined by extending the limiting stage along the axis of the mounting portion 23. The mounting portion 23 is provided with a plurality of fourth through holes 234, and the plurality of fourth through holes are disposed at intervals along a circumferential direction of the mounting portion 23. Each of the plurality of fourth through holes 234 penetrates through the distal end of the teeth portions 233, respectively, and extends to the limiting stage 232 and the base 231. Thus, the teeth portions 233 can extend and contract along a radial direction of a cross section of the mounting portion 23. Therefore, the plurality of fourth through holes 234 can make the teeth portions 233 disposed on the mounting portion 23 separate along the radial direction of the mounting portion 23, and further make the teeth portions 233 expand under an abutting force outwards along the radial direction of the mounting portion 233. The plurality of fourth though holes 234 will facilitate deformation of the mounting portion 23.

Referring to FIG. 9(a) to FIG. 9(c), FIG. 9(a) is a stereo structural schematic diagram of a first limiting portion in an embodiment; FIG. 9(b) is a schematic diagram of a first limiting portion in one of the use states in an embodiment; and FIG. 9(c) is a schematic diagram of a first limiting portion in one of the use states in an embodiment.

The first limiting portion 40b includes a first substrate 41b and a first protrusion 42b. The first substrate 41b is accommodated in the proximal end of the capsule 111. The first protrusion 42b extends out from the first substrate 41b to the side wall of the capsule 111, and is limited close to the limiting stage 232. In some embodiments, the number of the first protrusion 42b is two, and the two first protrusions 42b are connected to both ends of the first substrate 41b, respectively. In consideration of force balance of the first limiting portion 40b, the two first limiting stages 42b are symmetrical disposed on an axis of first limiting portion 40b, so as to better limit the limiting stage 232.

In some embodiments, an angle defined by the first substrate 41b and the first protrusion 42b is equal to or smaller than 30°, and the first substrate 41b can bend towards the proximal end of the first protrusion 42b or the distal end of the first protrusion 42b relative to the first protrusion 42b. In order to facilitate deformation of the first limiting portion 40b, the first substrate 41b and the first protrusion 42b are disposed in the same plane in some embodiments.

In consideration of deformation or out of operation of the first limiting portion 40b that make the processing unit 11 separate from the limiting stage 232 and release from the delivery pipe 21, the end surface of the proximal end of the first substrate 41b is provided with a deforming portion 41b1. The deforming portion 41b1 is configured to make the first substrate 41b deform or out of operation when the first substrate 41b is under an action of external force.

The deforming portion 41bl can be an opening hole or an opening groove disposed on the first substrate 41b. In some embodiments, the deforming portion 41b1 is groove-shaped penetrating through an end surface of the proximal end of the first substrate 41b. In some embodiments, the deforming portion 41b1 is located on the center axis of the first substra53 41bl, and perpendicular to the axes of the two first protrusions 42b. It should be noted that the deforming portion 41b1 can be disposed at other positions on the end surface of the proximal end of the first substrate 41b, and the deforming portion 41b can be not a through hole or groove-shaped. On the premise of not affecting deformation or out of operation of the first limiting portion 40b, the structure and position of the deforming portion 41b1 is not limited in the present invention.

A second through hole 41b0 is defined at a center of the first substrate 41b, and the distal end of the manipulation member 22 penetrates through the second through hole 41b0 and combine with the processing unit 11 to drive the processing unit 11 to simultaneously move. The distal end of the manipulation member 22 does not move in the second through hole 41b0. The distal side of the manipulation member 22 is not in a round-shaped, and the cross section of the distal end of the manipulation is approximately in a rectangle shape, and the proximal end of the manipulation member is in a column shape, thereby facilitating simultaneous move of the processing unit 11 and the manipulation member 22 in the delivery pipe 21, and not affecting the un-loaded processing unit.

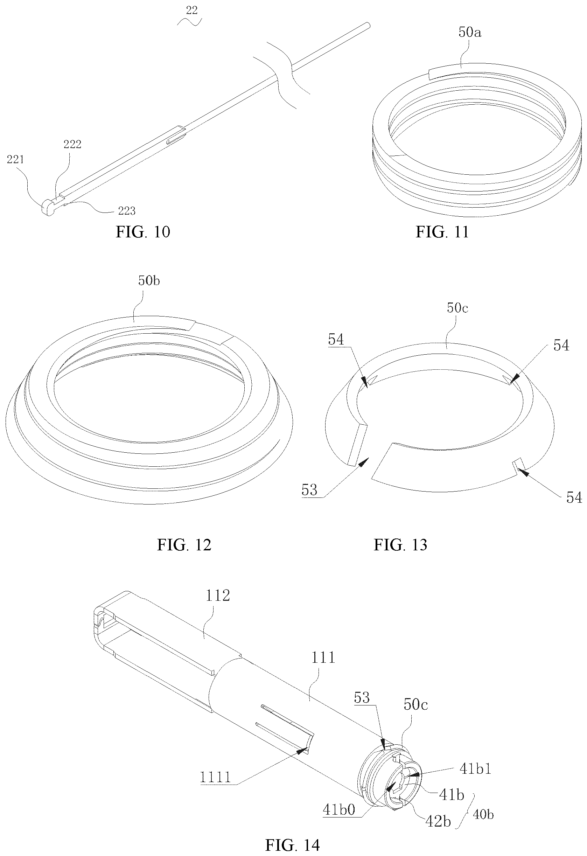

Referring to FIG. 10, FIG. 10 is a structural diagram of a manipulation member 22 in an embodiment. The distal end of the manipulation member 22 can include a head portion 221, a concave portion 222 and a shoulder portion 223 from the distal end of the manipulation member 22 to the proximal end of the manipulation member 22, which facilitate the manipulation member 22 driving the distal processing unit 11 to simultaneously move. Outer diameters of members of the manipulation member 22 except for the head portion 221 are less than the inner diameter of a round-shape hole.

In order to facilitate penetrating of the manipulation member 22 from the proximal side to the distal side of the first substrate 41b and maintain the initiating effect of the first limiting portion 40b, and improving the assembly efficiency, a shape of the second through hole 41b0 matches with the distal end of the manipulation member 22. A minimum inner diameter of the second through hole 22 is smaller than a maximum outer diameter of the head portion 221. The second through hole 41b0 is proximately in rectangle shape, and two long edges of a cross section of the second through hole 41b0 extends outwards, respectively, and define a round-shaped hole. A diameter of the round-shaped hole is greater than the outer diameter of the proximal end of the manipulation member 22, so that the proximal end of the manipulation member 22 can rotate in the second through hole 41b0. The diameter of the round-shaped hole is smaller than the maximum diameter of the head portion 221, so as to limit the head portion 221 to move towards the proximal end of the first substrate 41b and rotate in the second through hole 41b0. When the multiple processing device is assembled, the head portion 221 penetrates through the rectangle-shaped hole from the proximal side of the first substrate 41b to the distal side of the first substrate 41b and rotate the manipulation member 22. Outer diameters of all components in the manipulation member 22 except for the head portion 221 are smaller than the inner diameter of the round-shaped hole, so that the head portion can penetrate through the rectangle-shaped hole. After the head portion 221 is rotated at a certain angle, the head portion 221 can abut against the circumference wall of the second through hole 41b0, and cannot be drawn back through the second through hole 41b0. It should be noted that "drawn back" means the head portion 221 is drawn along a direction opposite to the direction the head portion 221 penetrates through the second through hole 41b0.

Referring to FIG. 11 to FIG. 13, a second limiting portion 50 in different embodiments is shown. In the embodiment of FIG. 11, the second limiting portion 50a is a column-shaped spring. In the embodiment of FIG. 12, the second limiting portion 50b is a tower-shaped spring, and an outer diameter of the tower-shaped gradually decreases towards a direction of the distal end of the delivery pipe 21. In the embodiment of FIG. 13, the second limiting portion 50c is provided a first opening 53, which extends along an axis of the second limiting portion 50c and provides a deformation allowance facilitate an elastic deformation of the second limiting portion 50c the radial direction of the second limiting portion 50c.

Referring to FIG. 12 and FIG. 13, an inner diameter of the second limiting portion 50b or 50c gradually decreases along a direction from the proximal end of the delivery pipe 21 to the distal end of the delivery pipe 21.

In some embodiments, the second limiting portion 50 is disposed at the distal end of the delivery pipe 21, and accommodated in the proximal end of the limiting stage 232. When the second limiting portion 50 of the at least one processing units 11 is in a state without radial force, an inner diameter of a distal end of the second limiting portion 50 is smaller than an outer diameter of the at least two processing units 11, and an inner diameter of a proximal end of the second limiting portion 50 is larger than the outer diameter of the at least one processing units 11.

In some embodiments, the side wall of the second limiting portion 50 is provided with a first opening 53 providing a deformation allowance, and the first opening 53 penetrates through the side wall of the second limiting portion50. And/or, the side wall of the second limiting portion 50 is further provided with a second opening 54, which extends from the proximal end of the side wall of the second limiting portion50 to the side wall of the second limiting portion 50. The number of the second opening 54 is multiple, and the plurality of second openings 54 are disposed at intervals along a circumference of the second limiting portion 50.

By designing the first opening and/or the second opening 54, a radial counter force suffered by the second limiting portion 50 when the distal end of the second limiting portion 50 penetrates through the limiting stage 232 can be reduced, so as to facilitate pushing the second limiting portion 50 from the delivery pipe 21. It should be noted that after the second limiting portion 50 is pushed out from the limiting stage 232, the distal end of the second limiting portion 232 will be restored by expanding along the radial direction of the distal end of the second limiting portion 50, so as to better abut against the distal end of the limiting stage 232.

Referring to FIG. 14, FIG. 14 is an assembly diagram of a processing unit 11, a first limiting portion 40b and a second limiting portion 50c in an embodiment. The first limiting portion 40b is accommodated in a proximal end of the capsule 111, so as to abut against a proximal end of the limiting stage 232. The second limiting portion 50c is sleeved on the proximal end of the capsule 111.

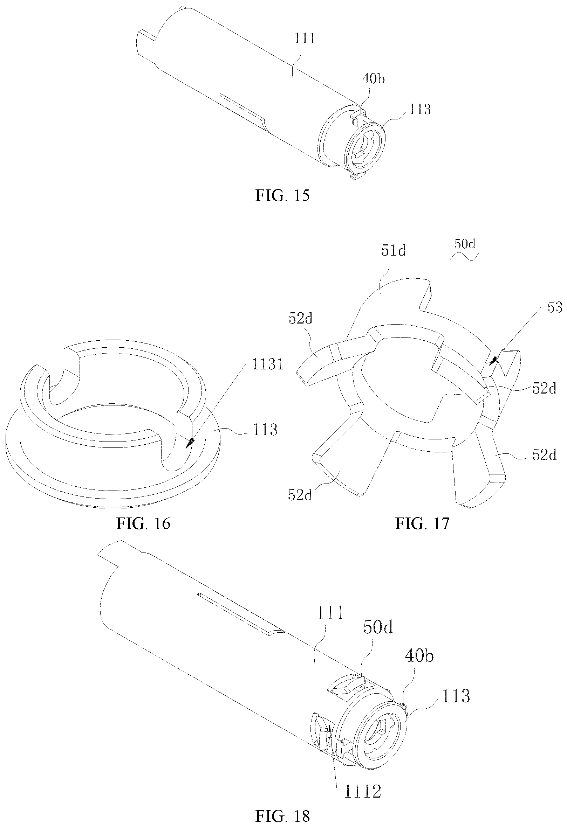

Referring to FIG. 15 and FIG. 16, FIG. 15 is an assembly diagram of a capsule 111 and a first limiting portion 40b in an embodiment; and FIG. 16 is a stereo structural schematic diagram of a pedestal 113 in an embodiment. In order to facilitate mounting the first limiting portion 40b, the processing unit 11 further includes a pedestal 113 fixed to the proximal end of the capsule 111. The pedestal 113 is provided with an accommodation chamber configured to accommodate the first substrate 41b, and a sidewall of the pedestal 113 is provided with a third through hole 1131 configured for the first protrusion 42b to extend outwards. In some embodiments, the pedestal 113 and the proximal end of the capsule 111 are fixed by welding.

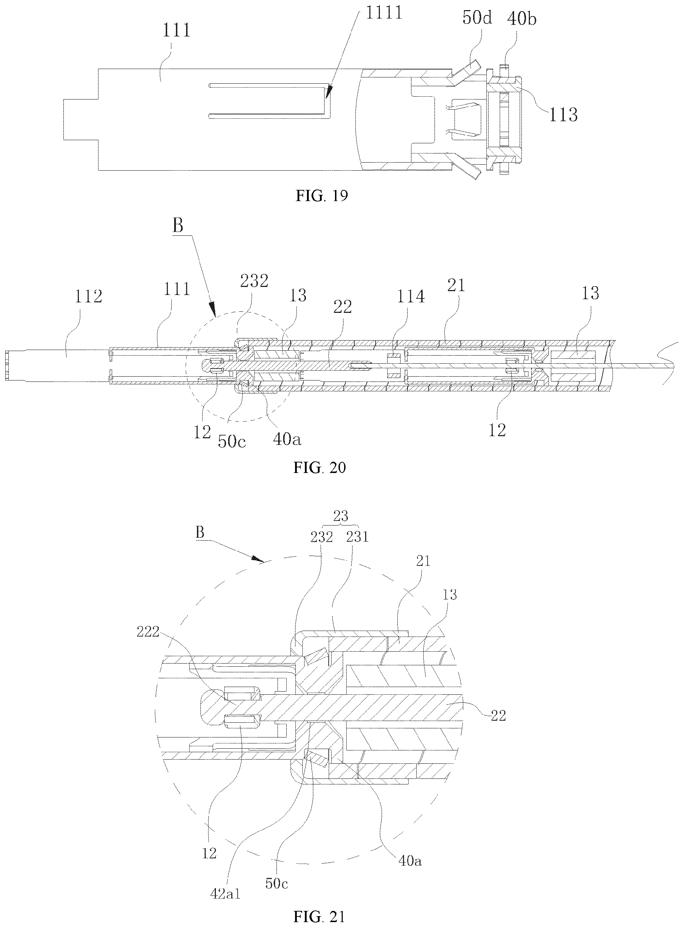

Referring to FIG. 17 to FIG. 19, FIG. 17 is a structural schematic diagram of a second limiting portion 50d in an embodiment; FIG. 18 is an assembly diagram of a processing unit 11, a first limiting portion 40b and a second limiting portion 50d in an embodiment; and FIG. 19 is a schematic diagram of FIG. 18 from another view angle.

When the second limiting portion 50d is disposed at a distal end of the limiting stage 232, the processing unit 11, the first limiting portion 40b and the second limiting portion 50c can have the structures shown in FIG. 14. The second limiting portion 50d includes a second substrate 51d and a second protrusion 52d. The second substrate 51d is accommodated in the capsule 111, and the second protrusion extends towards a sidewall of the capsule 111 and limits the limiting stage 232. The second substrate 51d is fixed to the inner wall of the capsule 111 by methods of welding, riveting, clamping, jointing and the like.

Furthermore, the second substrate 51d can be provided with a first opening 53, which facilitates the second limiting portion 50d to penetrate through the limiting stage 232. In some embodiment, the number of the second protrusion 52d is multiple, and the plurality of second protrusions 52d are disposed at interval along a circumference of the second substrate 51b. Accordingly, the capsule 111 is provided with a plurality of opening holes 1112 for the second protrusion 52d to extend out.

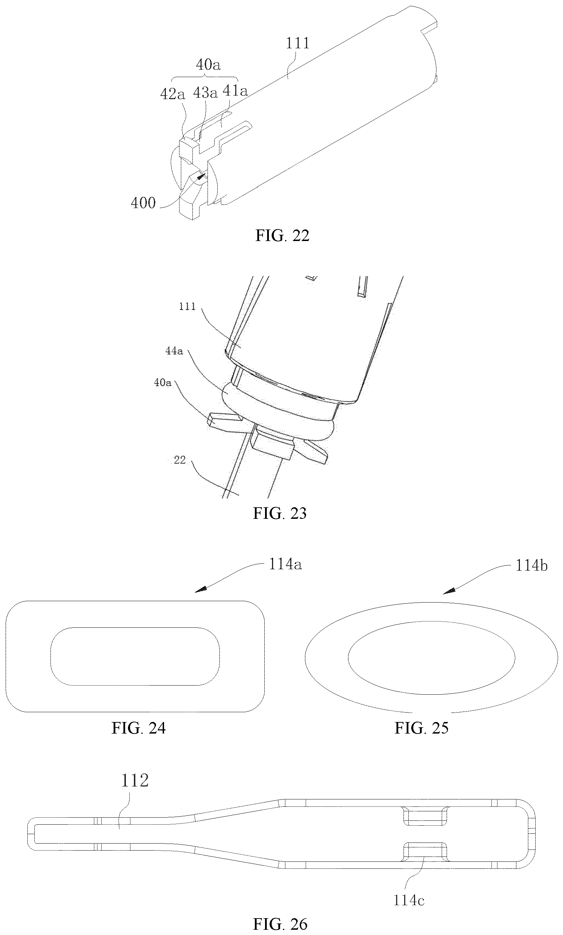

Referring to FIG. 20 to FIG. 22, FIG. 20 is a section view of a multiple processing device for an endoscope 100 in an embodiment; FIG. 21 is an enlarged view of portion B in FIG. 20; and FIG. 22 is an assembly diagram of a capsule 11 and a first limiting portion 40a in an embodiment.

In order to facilitate combination of the distal end of the manipulation member 22 with a first processing unit 11 closer to the operator after the distal processing unit 11 is released, and ensuring that each of the processing units 11 can be repeatedly opened and closed to effectively clamp the lesion, the first limiting portion 40a in some embodiments of the present invention is provided with a cavity 400 through which the manipulation member 22 penetrates at the inner side of the first limiting portion 40a. The distal end of the manipulation member 22 penetrates through the cavity 400 and connects to the processing unit 11. When the first limiting portion 40a abuts against the manipulation member 22, the first limiting portion 40a deforms and expands along a circumference direction of the capsule 111, so as to clip to the distal end of the delivery pipe 21. When the manipulation member 22 is driven towards the proximal end of the delivery pipe 21 by a relative large pulling force, the manipulation member 22 separates from the cavity 400, and the first limiting portion 40a contracts along the circumference of the capsule 111, so as to separate from the distal end of the delivery pipe 21. At this time, the capsule 111 and the first limiting portion 40a can release from the distal end of the delivery pipe 21.

In some embodiments, a cavity through which the manipulation member 22 penetrates through is defined in the center of the first limiting portion 40a. The distal end of the manipulation member 22 penetrates through the cavity 400 and connects to the processing unit 11, and the second limiting portion 50c is located at the distal end of the delivery tube 21, close to the proximal side of the limiting stage 232.

Furthermore, the first limiting portion 40a includes an extending portion 41a, a clipping portion 42a and a thin portion 43a. the extending portion is connected to the side wall of the capsule 111. The clipping portion 42a is connected to the proximal end of the extending portion 41a. The inner wall of the clipping portion 42a extends inwards and defines a clipping protrusion 42a1. When the manipulation member 22 abuts against the clipping protrusion 42a1, the clipping protrusion 42a1 deforms and expands along the radial direction of the capsule 111, so as to clip to the distal end of the second limiting portion 50c. The outer surface of the thin portion 43a abuts against the inner surface of the second limiting portion 50c. An end surface of the distal end of the clipping portion 42a abuts against an end surface of the proximal end of the second limiting portion 50c. An end surface of the extending portion 41a abuts against an end surface of the distal end of the second limiting portion 50c.

Therefore, on premise of not increasing the outer diameter of the capsule 111, the capsule 111 can be stably clipped to the distal end of the limiting stage 232. In addition, when the first limiting portion 40a and the manipulation member 22 are not combined, the clipping portion 42a is elastically contracted, and will not interfere with the delivery pipe 21, which facilitate freely delivery of the processing unit 11 in a narrow cavity of the delivery pipe 21 under conditions that a biopsy channel of the endoscope bends to an angle over 180°.

Furthermore, the proximal end of the extending portion 41a is level with the proximal end of the capsule 111, and a gap is located between the extending portion 41a and the side wall of the capsule 111. It should be understood that the extending portion 41a can be connected to the proximal end of the capsule 111.

An inner diameter at a proximal side of the center through hole of defined by the second limiting portion 50c is smaller than the outer diameter of the clipping portion 42a and is greater than or equal to the outer diameter of the capsule 111. At the same time, the inner diameter of the center through hole defined by the second limiting portion 50c decreases from the proximal end of the second limiting portion 50c to the distal end of the second limiting portion 50, so that a driving force driving the second limiting portion 50c to expand along a radial direction is generated when the processing unit 11 is driven towards the distal side. Therefore, after the capsule 111 penetrates through the second limiting portion 50c, the capsule 111 cannot move back and force along a length direction of the delivery pipe 21, so that the processing unit 11 is kept at the distal end of the delivery pipe 21 by the first limiting portion 40a and the second limiting portion 50c.

Furthermore, the inner diameter of the first through hole 230 is greater than the outer diameter of the distal end of the second limiting portion 50c, and smaller than the outer diameter of the proximal end of the second limiting portion 50c.

When the processing unit 11 is in the combined state, the distal end of the second limiting portion 50c abuts against a stepped face between a clipping portion 42a and the thin portion 43a. The proximal end of the limiting stage 232 abuts against the distal end of the first limiting portion 40a, and the manipulation member 22 is configured to make the clamping arm 112 move while switching between the opened state and the closed state. The outer diameter of the first limiting portion 40a is greater than a greater one of the diameter of the first through hole and the inner diameter of the second limiting portion 50.

When the processing unit 11 is in the separated state, the proximal end of the slidable portion 34 moves to release the combination between the manipulation member 22 and the processing unit 11 and the first limiting portion 40a. When the manipulation member 22 is driven by a relatively large driving force towards the proximal end of the delivery pipe 21, the manipulation member 22 separates from cavity 400. The first limiting portion 40a can contract along a radial direction of the capsule 111, so that the clipping portion 42a can separate from the distal end of the second limiting portion 50c, and the combination between the first limiting portion 40a and the limiting stage 232 is out of operation. The outer diameter of the first limiting portion 40a is smaller than a smaller one between the diameter of the first through hole 230 defined by the limiting stage 232 and the inner diameter of the second limiting portion 50. At this time, the capsule 111 and the first limiting portion 40a is released from the distal end of the delivery pipe 21.

The extending portion 41a and the inner wall of the capsule 111 can be formed via a integrally forming process; and optionally, can be separately formed, and then fixed to the inner wall of the capsule 111 by fixing methods such as screwing, welding, riveting and the like, which are not limited herein in the present invention.

In some embodiments of the present invention, the number of the first limiting portion is multiple, and the plurality of the first limiting portions 40a are centrally symmetric disposed relative to the side wall of the capsule 111. On premise of not influencing elastic expansion or elastic contracting of the first limiting portion 40a, the present invention will not limit the number and arrangement of the first limiting portion 40a.

In some embodiments, the plurality of first limiting portions 40a are disposed at intervals along the circumference of the capsule 111. In some embodiments, the number of the first limiting portion 40a is two, and are symmetrically disposed along the circumference of the capsule 111. It should be understood that in some embodiments, the number of the first limiting portion 40a can be larger than or equal to three. On premise of not influencing elastic expansion or elastic contracting of the first limiting portion 40a, the present invention will not limit the number and arrangement of the first limiting portion 40a.

Referring to FIG. 23, FIG. 23 is an assembly diagram of a capsule 111 and a first limiting portion 40a in an embodiment. In order to facilitate elastic retracting of the first limiting portion 40a when the manipulation member 22 is separated, the processing unit 11 further includes an elastic member 44a sleeved on the thin portion 43a. An inner surface of the elastic member 44a abuts against an outer surface of the thin portion 43a.

By designing the thin portion 43a on the first limiting portion 40a, a diameter of the first limiting 40a can be relatively small. Thus, the first limiting portion 40a will not interfere with or clip to the inner wall of the delivery pipe 21, which facilitate freely delivery of the processing unit 11 in a narrow cavity of the delivery pipe 21 under conditions that a biopsy channel of the endoscope bends to an angle over 180°.

In some embodiments, the elastic member 44a is preferably but not limited to an elastic ring. When the manipulation member 22 penetrate through a cavity 400 and connects to the processing unit 11, an inner wall of a clipping protrusion 42a1 abuts against the outer surface of the manipulation member 22, and both the first limiting portion 40a and the elastic member 44a are in an elastic expansion state. When the manipulation ember 22 separate from the processing unit 11 and release from the cavity 400, the thin portion 43a contracts inwards along a radial direction of a cross section of the thin portion 43a under the action of an elastic force of the elastic member 44a, so as to facilitate the processing unit 11 penetrating through the limiting stage 232. It should be understood that the first limiting portion 40a can be made of an elastic material and contracts inwards along a radial direction of the limiting portion 40a on the basis of the resilience of the elastic material.

Furthermore, in order to ensure stable combination between the manipulation member 22 and a first processing unit 11 closer to the operator, the processing unit 11 further includes a limiting member 114 (as shown in FIG. 20) accommodated in the clamping arm 112. When the processing unit 11 is in the closed state, the manipulation member 22 penetrates through the limiting member 114 and is limited by the limiting member 114, so that the head portion 221 abuts against the clamping member 12 towards the proximal side. Thus, when the manipulation member 22 move towards the proximal end of the delivery pipe 22, the manipulation member 22 can rotate in the clamping arm 112 along the circumference of the manipulation member. In some embodiments, the limiting member 114 is approximately in tube-shaped, and a cross section of the limiting member 114 matches with an outer side of the manipulation member 22. A maximum outer diameter of the limiting member 114 is smaller than a minimum gap between the two clamping arms 112 when the processing unit 11 is in the closed state.

Referring to FIG. 24 to FIG. 25, FIG. 24 and FIG. 25 are schematic diagrams of the limiting portion 114 in different embodiments. FIG. 24 is a limiting member 114a having a cross section in a rectangle-shaped; and FIG. 25 is a limiting member 114b having a cross section in an oval-shaped.

After a distal processing unit 11 is released, the manipulation member 22 is driven towards the proximal end of the delivery pipe 21. Before the distal end of the manipulation member 22 and a first processing unit 11 closer to the handle portion is effectively combined, free rotation of the manipulation member 22 in the delivery pipe 21 will affect combination of the distal end of the manipulation member 22 and the first processing unit 11 closer to the handle portion. Therefore, the manipulation member 22 can only move relative to the axis of the delivery pipe 21 under the action of the limiting member 114, until the manipulation member 22 is combined with the first processing unit 11 closer to the handle portion. When the clamping arms 112 of the first processing unit 11 closer to the handle portion are in the opened state, the limiting member 114 can fall into the living body from the processing unit 11.

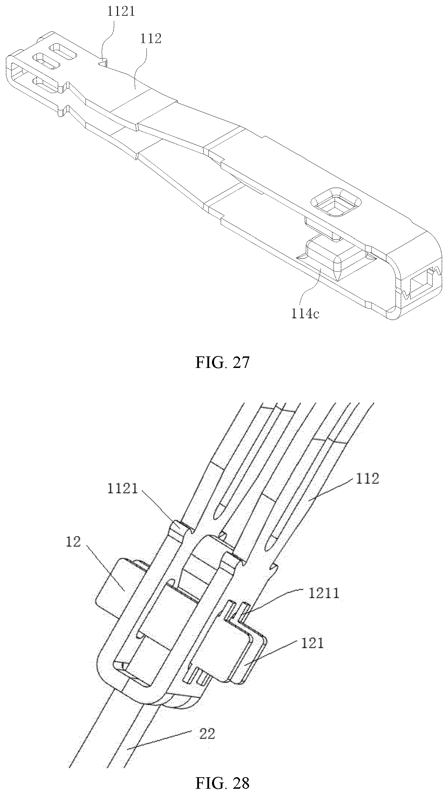

It should be understood the limiting member 114 and the clamping arm 112 can be separately disposed, or can be formed via an integrally forming process. In some embodiments, such as the embodiments shown in FIG. 26 and FIG. 27, a limiting member 114c and the clamping arm 112 are formed via an integrally forming process. The limiting portion 114c is a protrusion portion disposed on an inner wall of the clamping arm 112, and the protrusion portion is defined by protruding a part of the inner wall of one of the clamping arms 112 towards the other clamping arm 112. The protrusion portion can reduce a gap between the two clamping arms 11, and make the gap between the limiting members 114c smaller than the maximum outer diameter of the manipulation member 22, so as to limit a circumference rotation of the manipulation member 22 relative to the clamping arm 112.

Referring to FIG. 28, FIG. 28 is an assembly diagram of a clamping member 12, a processing unit 11 and a manipulation member 22 in an embodiment. The processing unit 11 includes two opposite clamping arms 112 disposed at interval. The processing unit 11 includes a clamping member 12 disposed between the two clamping arm 112. A concave portion 222 is fixed between the two clamping arms 112, the clamping member 12 abuts against the proximal end of the head portion 221 and the distal end of the shoulder portion 223. Under a preset force, the clamping member 12 can deform outwards or be out of operation to release the combination between the concave portion 222 and the two clamping arms 112. When the clamping member 12 suffers from a force greater than the preset value, a joint between the clamping member 12 and the concave portion 222 slightly deforms or cracks, and the manipulation member 22 separates from the processing unit 11. Furthermore, a distal end of the shoulder portion 223 inclines towards an axis of the shoulder portion 223, so as to ensure the manipulation member 22 clamping to the proximal end of the clamping member 12, thereby driving the processing unit 11 to rotate.

In some embodiments, the clamping member 12 includes two pins 121, and both of the two pins 121 penetrate through the two clamping arms 112. The two pins 121 can abut against two end surfaces of the concave portion 222 and limit the manipulation member 22. In the present embodiment, the tow pins 121 can have a separated structure.

Furthermore, the two pins 121 penetrate through the two clamping arms 112. The two pins 121 include a first end and a second end. The first ends of the two pins are provided with a limiting strip 1211 configured to limit the two pins 121 and fix the first end of the two pins to the clamping arm 112. The second ends of the two pins are free end. Therefore, when the clamping member 12 suffers from a force, the free ends of the two pins can move towards the first ends of the two pins to provide a deformation allowance, so as to facilitate separation between the manipulation member 22 and the clamping member 12.

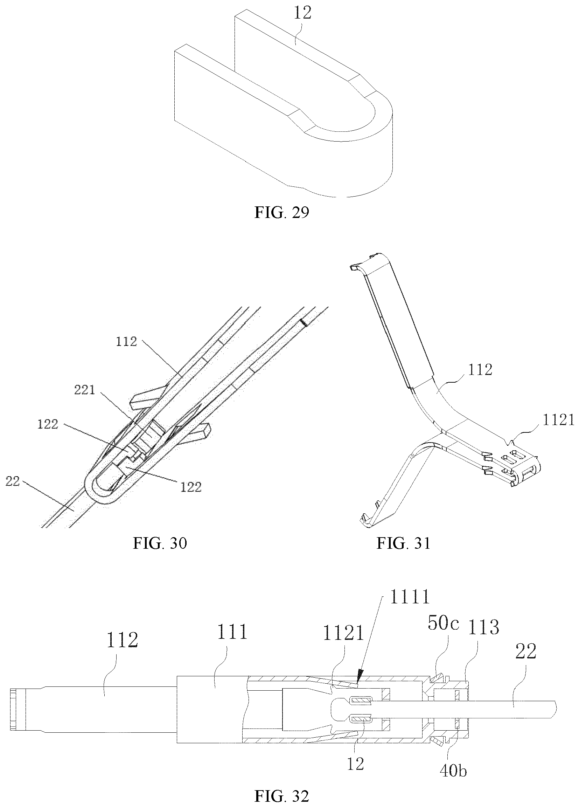

Referring to FIG. 29, FIG. 29 is a structural schematic diagram of a clamping member in an embodiment. In the embodiment, the two pins are in a U-shaped via an integrally forming process. The U-shaped pins can facilitate mounting the clamping member 12 on the clamping arm 112.

Referring to FIG. 30, FIG. 30 is an assembly diagram of a clamping member 12, a processing unit 11 and a manipulation member 22 in an embodiment. The clamping member 12 includes a folded portion defined by extending the clamping arm 112 towards the manipulation member 22. In some embodiments, side walls of each of the at least one clamping arm 112 extends towards the two concave portions 222 and define the folded portion 122. The folding portions 122 disposed on the two clamping arms 112 cooperate and surround with the two clamping arms 112 to abut against two end surfaces of the concave portion 222. In some embodiments, the folded portion 122 and the clamping arm 112 are formed via an integrally forming process. The two folded portions 122 disposed on each of the two clamping arms 112 is defined by extending a middle part of the clamping arm towards the concave portion 222.

Furthermore, consider that the processing unit 11 should be released from the distal end of the delivery pipe 21 in a clamping state after the process unit 11 clamps a target tissue, the processing unit 11 should have a self-locking function so as to lock the clamping arm 112 in the capsule 111. A proximal end of the clamping arm 112 is movably accommodated in the capsule 111. In the process of pushing the processing unit 11 from the delivery pipe 21 by the manipulation member 22, the clamping arm 112 can move relative to an axis of the capsule 111, so as to adjust the distance and angle between the clamping arm 112 and the target tissue, and an opening angle of the clamping arm 112. After the clamping arm 112 clamps the target tissue, by controlling the manipulation member 22 to the proximal end of the clamping arm 112 and driving the clamping arm 112 to simultaneously move, the clamping arm 112 is accommodated in and locked in the capsule 111, and the clamping arm 112 is closed.

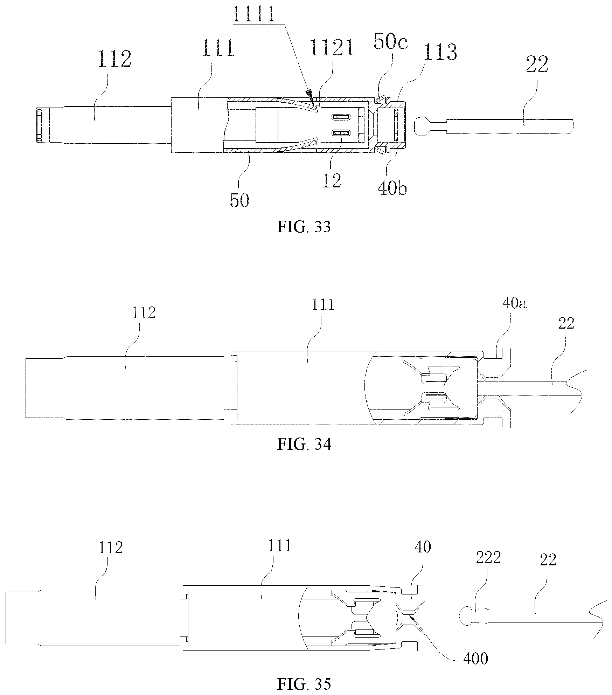

Referring to FIG. 31 to FIG. 33, FIG.31 is a structural schematic diagram of a clamping arm 112 in an embodiment; FIG.32 is a structural schematic diagram of a clamping arm 112 in a free state in an embodiment; and FIG.33 is a structural schematic diagram of a clamping arm 112 in a self-locked state in an embodiment.

In the present embodiment, a part of a sidewall of the proximal end of the clamping arm protruding outwards is defined as a locking portion, a part of a sidewall of the capsule 111 bending inwards is defined as a clamping hole portion 1111, and the clamping hole portion 1111 matches with the locking portion 1121 and is capable of limiting the clamping arm 112 from moving towards a distal end of the capsule 111. When the clamping arm 112 is in a free state, the manipulation member 22 can penetrate through the capsule 111 and connect to the clamping arm 112. At this time, the clamping arm 112 can move relative to the axis of the capsule 111. When the clamping arm 112 is in a self-locked state, the manipulation member 22 is separated from the clamping arm 112, and the distal end of the clamping arm 112 is contracted and locked in the capsule 111.

Referring to FIG. 34, FIG.34 is a structural schematic diagram of a clamping arm 112 in a free state in an embodiment. In the present embodiment, by fixing a locking seat 1122 matching with the proximal end of the clamping arm 112 in the inner wall of the capsule 111, when the manipulation member 22 is operated at the proximal end side, the clamping arm 112 can be self-locked by driving the proximal end of the clamping arm 112 to clip to the locking seat. The locking seat and the inner wall of the capsule 111 can be formed integrally, or can be formed separately. It should be noted that on premise of not affecting self-locking effect of the clamping arm 112, the method for self-locking the clamping arm 112 is not limited in the present invention.

Referring to FIG. 20, a separating member 13 is disposed between adjacent two processing units 11 of the at least one processing units 11. The separating member can make the adjacent two processing units separate from each other, and is sleeved on the manipulation member 22. Therefore, each of the at least one processing units 11 are separated from each other. On one hand, it can prevent the at least one processing units 11 from interfering with each other and damaging each other; on the other hand, the separating member 13 can prevent a first processing unit 11 closer to the handle portion from unduly exposing when a second processing unit is released, and causing the end surface of the capsule 111 cannot be accommodated in the delivery pipe 21, thereby leading to the device out of operation. It should be noted that an inner diameter of the separating member 13 is larger than the outer diameter of the manipulation member 22. Therefore, the manipulation member 22 can be successfully transferred to the next processing unit 11 and control the next processing unit.

Furthermore, for a multiple processing device for an endoscope in conventional art, a plurality of processing units can be accommodated in the delivery pipe, and the manipulation member can move forth or back to drive the processing units to switch between a combined state and a separated state. An operating component disposed at a proximal end of the multiple processing device includes a slidable portion combined with the proximal end of the manipulation member, and the slidable portion can drive the manipulation member to move. In some embodiments, after the processing unit at the distal end is released, by operating the slidable portion towards the proximal end and driving the manipulation member to move, the manipulation member is combined with a first processing unit closer to the handle portion, until the first processing unit is released. Then repeatedly move the slidable portion and repeat the operation to eh manipulation to release the rest processing units.

However, in actual use, a user uses one hand to operate the multiple processing device for the endoscope, and uses the other hand to operate the endoscope. Therefore, a moving trail s 1 of the slidable portion is limited by a space between the thumb and the index finger. In consideration of convenient, a moving trail s3 of the manipulation driven by the slidable portion is relatively limited. Therefore, for the processing unit close to the proximal end near the delivery pipe, it is hard to effectively combine with the manipulation member as it is located in s2 outside the moving trail of the manipulation member. Thus, the delivery pipe cannot accommodate many processing units, and further use of the multiple processing device for the endoscope is limited.

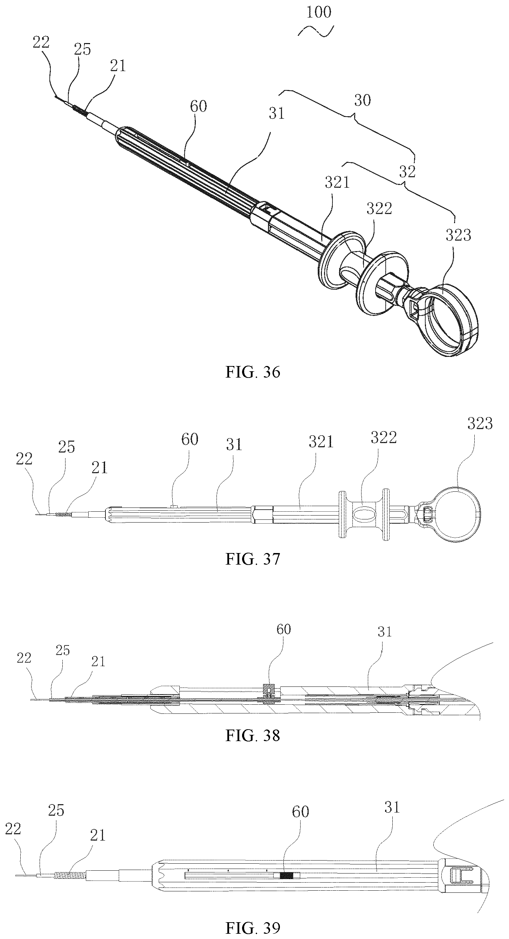

In view of the above, referring to FIG. 36 to FIG. 40, the present invention provides a multiple processing device for the endoscope 100, which is capable of accommodating a larger number of processing units 11 and enabling each of the at least two processing units 11 to be efficiently loaded.

The multiple processing device for an endoscope includes: a processing component 10, wherein the processing component 10 includes at least two processing units 11, and a proximal end of each of the at least two processing units 11 is provided with a first limiting portion that is capable of deforming or out of operation under an action of an external force. A delivery component 20, wherein the delivery component 20 includes a delivery pipe 21 accommodated in the processing component 10, a pushing pipe 25 penetrating through the delivery pipe 21 and an operating component penetrating through the pushing pipe 25, wherein a distal end of the pushing pipe 25 abuts against a proximal processing unit 11 of the at least two processing units 11. And an operating component, wherein the operating component includes a handle portion 31 combined with a proximal end of the delivery pipe 21. A slidable portion 32 combined with a proximal end of the operating component, which is configured to control a distal processing unit 11 of the at least one processing units 11 to open and close. And a pushing component 60 disposed on the handle portion 31, wherein the pushing component 60 includes a fixing seat 61 fixed to a proximal end of the delivery pipe 21. Wherein the handle portion 31 is provided with a sliding groove 312 for the fixing seat 61 to slide, and the handle portion 31 is provided with a marking portion configured for matching with the fixing seat 61 and showing the number of the at least two processing units 11.

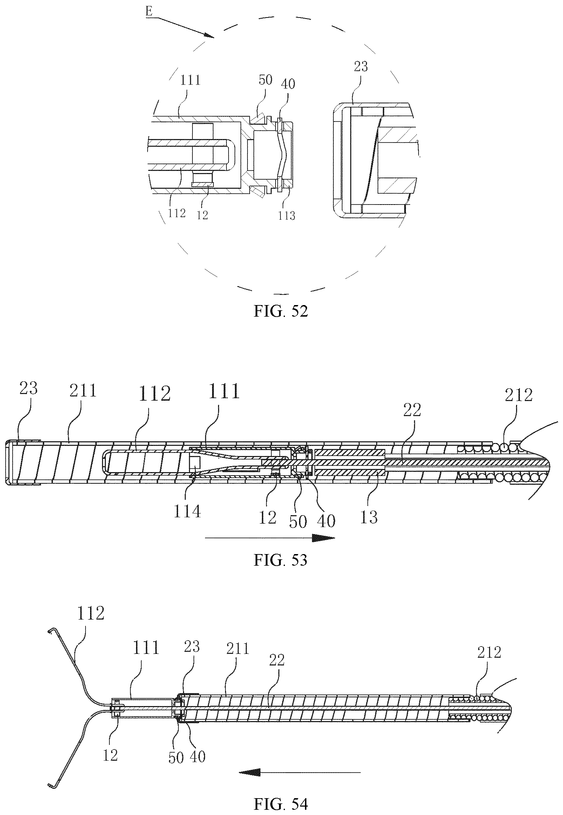

Furthermore, the pushing pipe 25 can penetrate through the delivery pipe 21 and the manipulation member 22 can penetrate through the pushing pipe 25. The material of the pushing pipe 25 includes at least one of PTFE (polytetrafluoroethylene), HDPE (high-density polyethylene), and Peek (poly(ether-ether-ketone)). The material can reduce the friction between the manipulation member 22 and the delivery pipe 21, so that the manipulation member 22 can move freely in and out of the delivery pipe 21. The distal end of the pushing pipe 25 is directly or indirectly through a separating member 10 (refer to FIG. 53) abuts against the proximal processing unit 11. In order to avoid bending due to a small diameter and a limited driving force of the pushing pipe 25 during a pushing process, it is possible to act indirectly on the processing component 10 by the separating member 10.

A processing unit 11 located on the proximal end of the delivery pipe 21 is able to move in a distal direction by pushing the pushing pipe 25 to move in the direction of a distal end of the delivery pipe 21, which enable the manipulation member 22 to effectively load and operate the processing unit 11 located outside of its loading stroke.

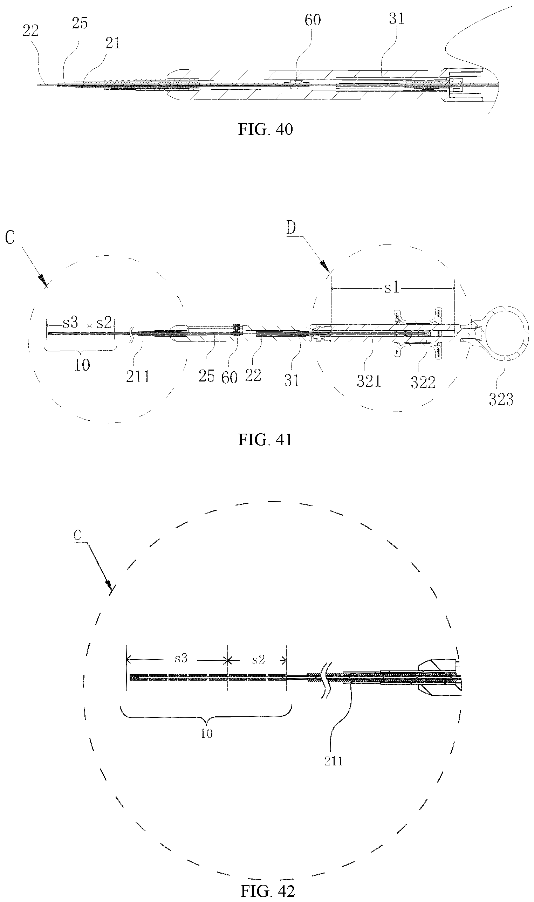

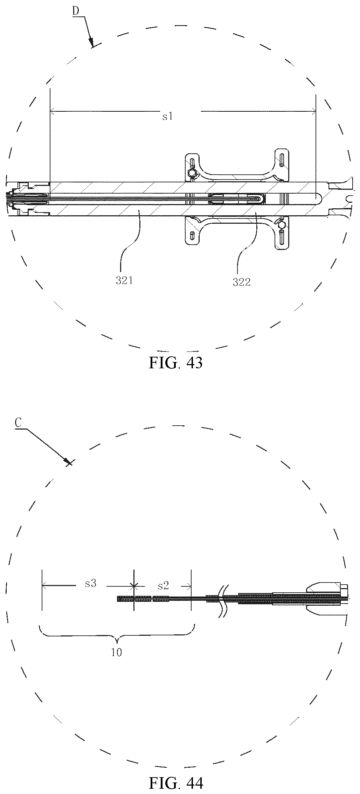

Referring to FIG. 41 to FIG. 44, in which s1 indicates a movement stroke of the slidable ring 322, and s3 indicates the stroke at which the manipulation member 22 can effectively load the processing unit 11, usually, s1 is equal to s3. s2 located proximal to s3 indicates that the manipulation member 22 cannot effectively load the processing unit 11 located within movement stroke of the slidable ring 322 by operating the slidable ring 322. In the present embodiments, each of the at least two processing units 11 located at s2 is able to be moved to s3 one by one by moving the pushing pipe 25 in the direction of a distal end of the delivery pipe 21

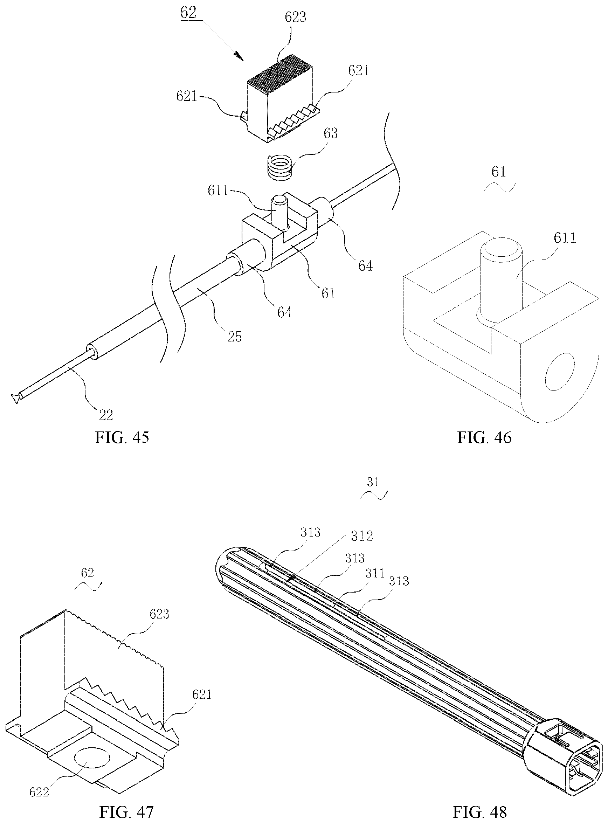

Referring to FIG. 45, in the present embodiments, the pushing component 60 includes a fixing seat 61 sheathed on the pushing pipe 25 and fixed to the pushing pipe 25. The fixing seat 61 is capable of driving the pushing pipe 25 to move relative to the delivery pipe 21 under an action of an external force. In this way, the at least two processing units 11 accommodated in the delivery pipe 21 can be effectively loaded by operating the manipulation member 22 by the slidable ring 322 and operating the pushing pipe 25 by the pushing component 60.

The fixing seat 61 is sheathed on the pushing pipe 25, and accordingly, the fixing seat 61 is provided with a shaft hole (not shown) for the pushing pipe 25 to penetrate through. In the present embodiment, the fixing seat 61 is fixed to the pushing pipe 25 by gluing, and in other embodiments, the fixing seat 61 is fixed to the pushing pipe 25 by riveting, welding, clamping and other means. And the fixing seat 61 can also be fixed to the outer wall of the pushing pipe 25. Without affecting the synchronous movement of the fixing seat 61 and the pushing pipe 25, this application does not limit the structure and arrangement position of the fixing seat 61.

Furthermore, the pushing component 60 further includes a locking member 62 and a resetting member, the locking member 62 is connected to the fixing seat 61, the resetting member is disposed between the fixing seat 61 and the locking member 62 and abuts against the locking member 62. The locking member 62 is provided with a first locking portion 621, the handle portion 31 is provided with a second locking portion 311, and the first locking portion 62 is capable of meshing with or separating from the second locking portion 311, so as to lock or unlock the locking member 62 with the handle portion 31.

When the locking member 62 is in a locked state with the handle portion 31, the pushing pipe 25 is unable to move. Since the distal end of the manipulation member 22 needs to operate the manipulation member 22 in a direction from the distal end of the delivery pipe 21 to the proximal end of the delivery pipe 21 when loading and combining with a corresponding processing unit 11, it is necessary to ensure that the processing unit 11 cannot move toward the proximal end of the delivery pipe 21, otherwise the manipulation member 22 and the processing unit 11 would move towards the proximal end of the delivery pipe 21 at the same time so that the processing unit 11 cannot be effectively loaded by the manipulation member 22. Therefore, in the present embodiments, when the pushing pipe 25 is in a locked state, the processing component 10 cannot move towards the proximal end of the delivery pipe 21 due to the restriction of the pushing pipe 25, so that effective loading of each of the at least two processing units 11 can be ensured in the locked state.

In the present embodiments, the resetting member is disposed between the fixing seat 61 and the locking member 62. A first end of the resetting member 63 abuts against the fixing seat 61, and a second end of the resetting member 63 abuts against the locking member 62, so that the locking member 62 always has a tendency to engage the second locking portion 311. It is understood that in other embodiments, the first locking portion 621 and the second locking portion 311 can also be locked by other means, such as clamping, magnetic suction, etc. Preferably, the first locking portion 621 on the locking member 62 is provided with a rack.

In the present embodiments, the locking member 62 and the handle portion 31 can be switched to an un-locked state by pressing the locking member 62 towards the fixing seat 61, at which time it is possible to move the locking member 62 in the direction of the axis of the delivery pipe 21 and drive the fixing seat 61 and the pushing pipe 25 to move simultaneously. After withdrawing the pressing pressure, the locking member 62 can move towards the handle portion 31 by the action of the resetting member 63 and restore the locked state with the second locking portion 311.

Furthermore, a plurality of the second locking portions 311 are fixedly arranged in the moving direction of the fixing seat 61 61 and is configured for a continuous rack. In the preferred embodiment, the stroke of the first locking portion 621 and the second locking portion 311 move through unlocking, moving and re-locking corresponding to the length of the processing unit 11.

Referring to FIG. 46 to FIG. 47, the fixing seat 61 is provided with a guiding column extending towards the locking member 62, the locking member 62 is provided with a guiding groove 622 through which the guiding column inserting, and the locking member 62 is capable of moving along an axis of the guiding column relative to the locking member 62. The movement of the locking member 62 is guided by the guiding column, so that it is smoother and more reliable to lock or unlock the locking member 62 with the handle portion 31.

The locking member 62 is further provided with an extension portion 623 stretching out from a sidewall of the handle portion 31 in a locked state, and the sidewall of the handle portion 31 is provided with a sliding groove 312, wherein the extension portion 623 is capable of stretching out from the sliding groove 312 and sliding along the sliding groove 312. In the preferred embodiment, the end face of the extension portion 623 is provided with a knurling (not shown) to increase a frictional force of the extension portion 623 and to facilitate the moving operation performed on itself.

In the preferred embodiment, the second locking portion 311 is provided on an inner wall of the handle portion 31, and the first locking portion 621 is disposed on an end face of the locking member 62 towards the second locking portion 311. It is understood that the second locking portion 311 may also be provided on the side wall of the sliding groove 312, and the first locking portion 621 is disposed on an end face of the locking member 62 towards the second locking portion 311.

Referring to FIG. 48, the handle portion 31 is provided with a marking portion 313 for ease of use, the number of the marking portion 313 arranged is one or more to mark the number of the remaining processing units in the delivery pipe 21. The arrangement and quantity of the marking portion 313 is adjusted according to the quantity of the processing units accommodated in the delivery pipe 21. Preferably, a plurality of marking portions 313 are disposed in equidistance along a moving direction of the fixing seat 61. Furthermore, the marking portion 313 is digital marking. It is understood that the marking portion 313 may also be other marking forms such as pattern marking.

In order to make the synchronous movement between the fixing seat 61 and the pushing pipe 25 more stable and reliable, the pushing component 60 further includes a crimper 64, which is sleeved on the pushing pipe 25 and fixed to the fixing seat 61. Preferably, the number of the crimper 64 is two, and the two crimpers 64 are fixed to two ends of the fixing seat 61, respectively.

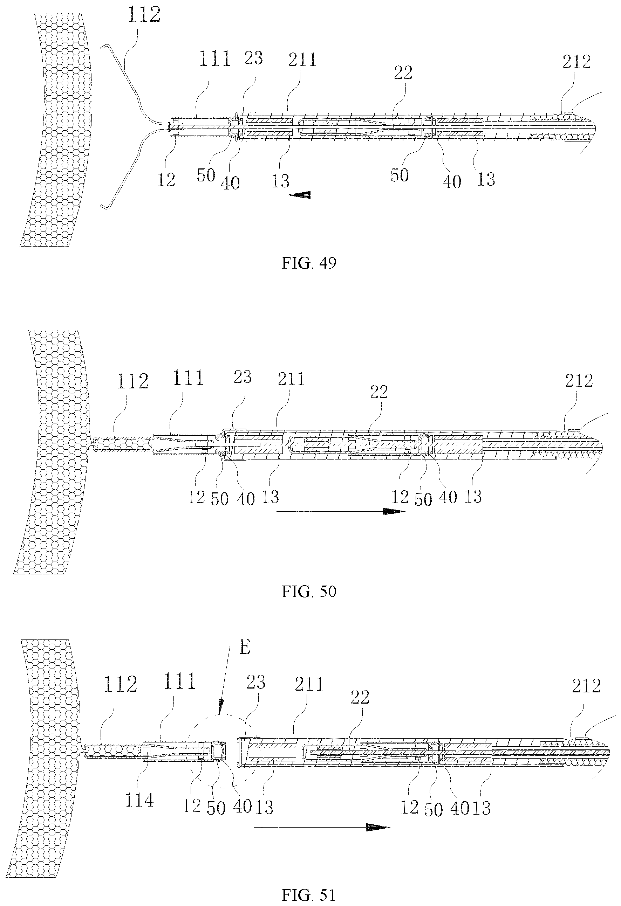

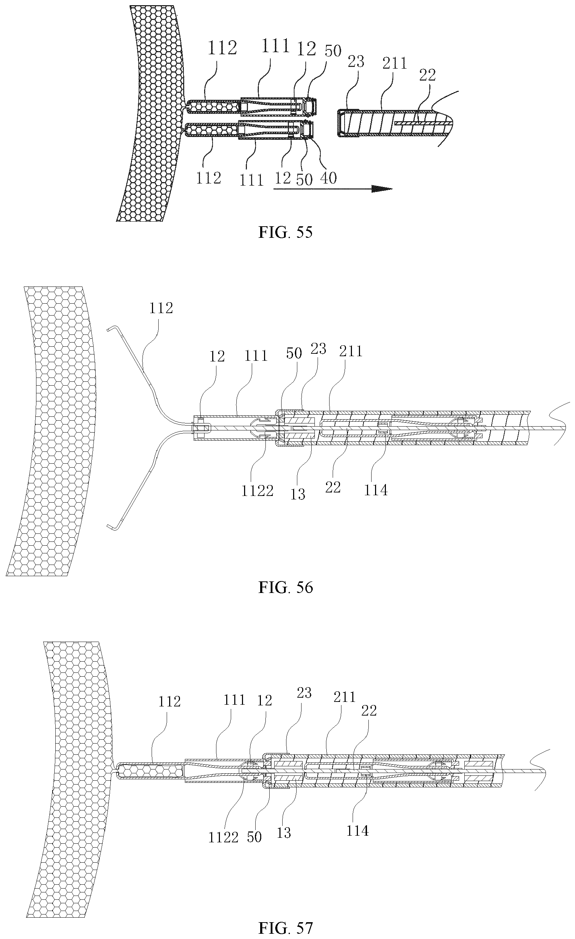

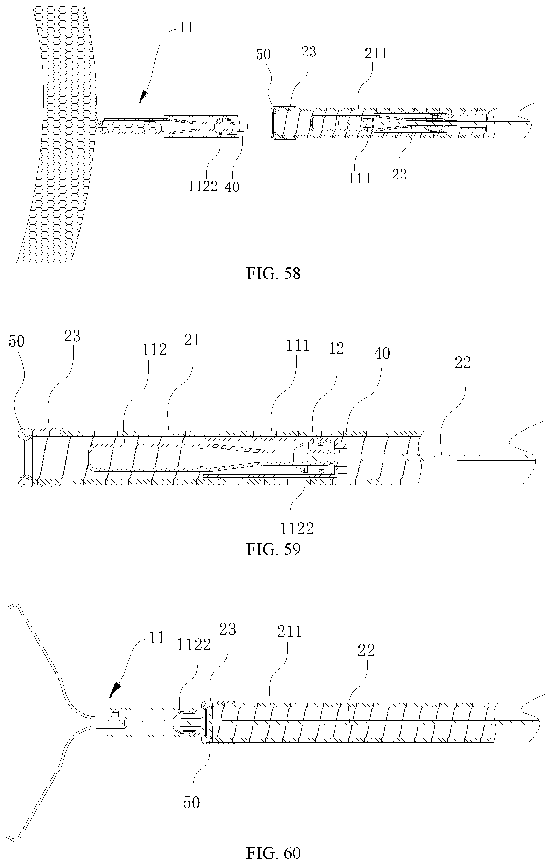

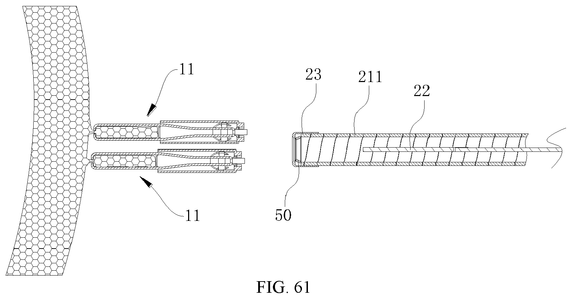

Referring to FIG. 49 to FIG. 61, FIGS. 49 to FIG. 55 show schematic diagrams in various states of use of the multiple processing device for endoscope provided in one embodiment of the present invention. FIGS. 56 to FIG. 61 show schematic diagrams in various states of use of the multiple processing device for endoscope provided in another embodiment of the present invention.

The method for use a multiple processing device for an endoscope includes the following steps. Step I, releasing the distal processing unit in the delivery pipe, and driving the manipulation member to move towards a proximal end of the delivery pipe, until it is impossible to drive the manipulation member further move towards a proximal end of the delivery pipe under a certain driving force, and a first user feedback, which shows the proximal processing unit is combined with a distal end of the manipulation member, is obtained. The first user feedback includes the sound of the manipulation member combined with the processing unit and the manipulation member cannot move further. At this point the manipulation member can drive the processing unit to move synchronously.

Step II, driving the manipulation member to move towards a distal end of the delivery pipe, until it is impossible to drive the manipulation member further move towards a distal end of the delivery pipe under a certain driving force, and a second user feedback, which shows the first limiting portion is clipped to the second limiting portion, is obtained. The second user feedback includes the sound of the first limiting portion is clipped to the second limiting portion and the manipulation member cannot move further. The manipulation member can drive the processing unit to move back and forth and rotate relative to the capsule, so as to perform repeated opening, closing and rotating operations on the processing unit until a clamping operation is completed by the processing unit.

Step III, after clamping tissue, driving the manipulation member in the direction of the proximal end of the delivery pipe, and when the first limiting portion is deformed (as shown in Figure 52) or out of operation, releasing the processing unit.

In some embodiments, the method for use a multiple processing device for an endoscope includes the following steps. Step I, moving the slidable portion in the direction of the distal end of the delivery pipe, so that a plurality of processing units located within the moving stroke of the slidable portion are driven by the manipulation member and released one by one.

Step II, pressing the pushing component and drive the pushing component to move towards the distal end of the delivery pipe so that a plurality of processing units located outside of the moving stroke of the slidable portion are driven by the pushing pipe and released one by one.

In some embodiments, the method for use a multiple processing device for an endoscope is based on the multiple processing device for an endoscope. The multiple processing device for the endoscope includes a processing component, a delivery component and a pushing component. The processing component includes at least two processing units including a proximal processing unit and a distal processing unit. The pushing component includes a delivery pipe configured for accommodating the processing component, a pushing pipe penetrating through the delivery pipe, and a manipulation member penetrating through the pushing pipe. A distal end of the pushing pipe abuts against the proximal processing unit, and the pushing component is fixed to a proximal end of the pushing pipe.

The method includes the following steps. Step I), releasing the distal processing unit in the delivery pipe, and driving the manipulation member to move towards a proximal end of the delivery pipe, until a first user feedback, which shows the first processing unit is combined with a distal end of the manipulation member, is obtained.

Step II, driving the pushing component to move towards a distal end of the delivery pipe, and driving the proximal processing unit to move towards a distal end of the delivery pipe via the pushing pipe fixed to the pushing component, until a second user feedback, which shows the first processing unit is combined with the distal end of the delivery pipe, is obtained.

Step III, after the second user feedback is obtained, driving the distal processing component with the operating component to freely open or closed to obtain an expected state.

Furthermore, the pushing component includes a fixing seat, a locking member and a resetting member. The resetting member is disposed between the fixing seat and the locking member and abuts against the fixing seat and the locking member. The locking member is provided with a first locking portion matching with a second locking portion disposed on the handle portion. The step of driving the pushing component to move towards a distal end of the delivery pipe, and driving the proximal processing unit to move towards a distal end of the delivery pipe via the pushing pipe fixed to the pushing component. Until a second user feedback, which shows the proximal processing unit is combined with the distal end of the delivery pipe, is obtained further includes the following steps. When the pushing pipe is moved, driving the pushing component to the distal side relative to the delivery pipe, pressing the locking member to make the first locking portion and the second locking portion being unlocked. After the second user feedback is obtained, releasing the locking member to make the first locking portion and the second locking portion being locked.

In the present embodiments, the method for use a multiple processing device for an endoscope includes the following steps. Step I, releasing the distal processing unit in the delivery pipe, driving the manipulation member to move towards a proximal end of the delivery pipe, until a first user feedback, which shows a first processing unit is combined with a distal end of the manipulation member, is obtained.

Step II, driving the pushing component to move towards a distal end of the delivery pipe. When the pushing pipe is moved, pressing the locking member to make the first locking portion and the second locking portion being unlocked. After the second user feedback is obtained, releasing the locking member to make the first locking portion and the second locking portion being locked.

Step III, after the second user feedback is obtained, driving the distal processing component with the operating component to freely open or closed to obtain an expected state.

Similarly, a plurality of processing units accommodated in the delivery pipe can be released one by one. The multiple processing device for endoscope is able to be inserted once to release multiple processing units for clamping the lesion, which greatly reduces the operating time and discomfort during a surgery.

The technical features of the above-described embodiments may be combined in any combination. For the sake of brevity of description, not all possible combinations of the technical features in the above embodiments are described. However, as long as there is no contradiction between the combinations of these technical features, all should be considered as within the scope of this invention.

The above-described embodiments are merely illustrative of several embodiments of the present invention, and the description thereof is relatively specific and detailed, but is not to be construed as limiting the scope of the invention. It should be noted that the number of variations and modifications may be made by those skilled in the art without departing from the spirit and scope of the invention. Therefore, the scope of the invention should be determined by the appended claims.

## Claims

1. A multiple processing device for endoscope, comprising:
at least two processing units, wherein the processing units comprises a capsule and a clamping arm, the clamping arm is accommodated in the capsule and has an opened state and a closed state, the clamping arm is capable of switching between the opened state and the closed state, and a proximal end of the capsule is provided with a first limiting portion that is capable of deforming or being out of operation under an action of an external force;
a delivery component, wherein the delivery component comprises a delivery pipe for accommodating the processing units, a distal end of the delivery pipe is provided with a limiting stage protruding towards an axis of the delivery pipe, and a manipulation member penetrating through the delivery pipe, and the manipulation member is capable of driving a distal processing unit of the processing units to switch from a combined state to a separated state, wherein in the combined state, the distal processing unit of the processing units is combined with the distal end of the delivery pipe, and in the separated state, the distal processing unit is separated from the distal of the delivery pipe; and
an operating component, wherein the operating component comprises a handle portion combined with a proximal end of the delivery pipe, and a slidable portion sleeved on the handle portion and combined with a proximal end of the manipulation member, and the slidable portion is configured to drive the manipulation member to move,
wherein the distal end of the delivery pipe and/or the proximal end of the capsule are further provided with a second limiting portion abutting against with the limiting stage and being capable of contracting or expanding along a radial direction of a cross section of the delivery pipe.

2. The multiple processing device for the endoscope of claim 1, wherein the second limiting portion is disposed at the distal end of the delivery pipe, in the combined state, the second limiting portion is located at a proximal end of the limiting stage, the first limiting portion is provided with a thin portion closed to an axis of the capsule, and the thin portion is configured to abut against the second limiting portion and limit the capsule.

3. The multiple processing device for the endoscope of claim 2, wherein in the separated state, an outer diameter of the first limiting portion is smaller than a smaller one selected from a diameter of a first through hole defined by the limiting stage and an inner diameter of the second limiting portion.

4. The multiple processing device for the endoscope of claim 2, wherein the first limiting portion is provided with an extending portion for accommodating the second limiting portion and an elastic member sleeved on a proximal end of the extending portion, and an inner surface of the elastic member abuts against an outer surface of the extending portion and is configured to converge the thin portion.

5. The multiple processing device for the endoscope of claim 1, wherein the first limiting portion comprises a first substrate and a first protrusion, the first substrate is accommodated in the proximal end of the capsule, and the first protrusion is connected to the first substrate and protrudes outwards, and the first protrusion protrudes towards a sidewall of the capsule and is limited near the limiting stage.

6. The multiple processing device for the endoscope of claim 5, wherein an angle defined by the first substrate and the first protrusion is equal to or smaller than 30°, and an end surface of a proximal end of the first substrate is provided with a deforming portion, which is configured to make the first substrate deform or be out of operation under the external force.

7. The multiple processing device for the endoscope of claim 6, wherein the deforming portion penetrates through the end surface of the proximal end of the first substrate.

8. The multiple processing device for the endoscope of claim 5, wherein a distal end of the manipulation member expanding is defined as a head portion, a second through hole is defined at a center of the first substrate, and the head portion is capable of penetrating through the second through hole, and a minimum inner diameter of the second through hole is smaller than a maximum outer diameter of the head portion.

9. The multiple processing device for the endoscope of any one of claims 5 to 8, wherein the processing unit further comprise a pedestal, which is fixed to the proximal end of the capsule, the pedestal comprises an accommodation chamber configured to accommodate the first substrate, and a sidewall of the pedestal is provided with a third through hole configured for the first protrusion to extend outwards.

10. The multiple processing device for the endoscope of any one of claims 1 to 8, wherein in the combined state, the first limiting portion is located in the limiting stage and clipped to the limiting stage, in the separated state, the first limiting portion is deformed or out of operation under the action of the manipulation member and is unclipped from the limiting stage, a diameter of the first through hole defined by the limiting stage is greater than an outer diameter of a distal end of the first limiting portion, and smaller than a diameter of a proximal end of the second limiting portion.

11. The multiple processing device for the endoscope of claim 2, wherein the inner diameter of the second limiting portion decreases from the proximal end of the delivery pipe to the distal end of the delivery pipe.

12. The multiple processing device for the endoscope of claim 11, wherein the second limiting portion is disposed at the distal end of the delivery pipe, and accommodated in the proximal end of the limiting stage, under conditions without radial force, an inner diameter of a distal end of the second limiting portion is smaller than an outer diameter of the processing units, and an inner diameter of a proximal end of the second limiting portion is larger than the outer diameter of the processing units.

13. The multiple processing device for the endoscope of claim 2, wherein in the combined state, a distal end of the second limiting portion abuts against the thin portion, and the proximal end of the limiting stage abuts against a distal end of the first limiting portion, the manipulation member is configured to make the clamping arm move while switching between the opened state and the closed state; and
in the separated state, a proximal end of the slidable portion is capable of moving to release a combination between the manipulation member, the processing units and the first limiting portion, so that a combination between the first limiting portion and the limiting stage is out of operation.

14. The multiple processing device for the endoscope of claim 2, wherein the second limiting portion is disposed at the distal end of the delivery pipe, and the second limiting portion comprises a plurality of teeth portions which are disposed at intervals along a circumference of the delivery pipe and gathered towards the distal end of the delivery pipe, distal ends of the teeth portions are configured to abut against the thin portion, and proximal ends of the teeth portions are configured to abut against the expanded first limiting portion.

15. The multiple processing device for the endoscope of claim 1, wherein the second limiting portion comprises a second substrate and a second protrusion, the second substrate is accommodated in the capsule, the second protrusion is connected to the second substrate and extended outwards, and the second protrusion is extended towards a sidew
all of the capsule and limited to the limiting stage.

16. The multiple processing device for the endoscope of claim 2, wherein the number of the second protrusion is multiple, and the second protrusions are disposed along at intervals a circumference of the second substrate.

17. The multiple processing device for the endoscope of any one of claims 11 to 16, wherein a sidewall of the second limiting portion is provided with a first opening which is capable of providing a deformation allowance, and the first opening penetrates through the sidewall of the second limiting portion; and/or,
the sidewall of the second limiting portion is further provided with a second opening extending from an end surface of a proximal end of the sidewall of the second limiting portion to the sidewall of the second limiting portion, the number of the second opening is multiple, and a plurality of second openings are disposed at intervals along a circumference of the second limiting portion.

18. The multiple processing device for the endoscope of claim 1, wherein a distal end of the manipulation member comprises a head portion, a concave portion and a shoulder portion; and
a proximal end of the clamping arm is provided with a clamping member through which the concave portion penetrates, and the clamping member abuts against a proximal end of the head portion and a distal end of the shoulder portion, respectively, and the clamping member is out of operation under a predetermined force.

19. The multiple processing device for the endoscope of claim 18, wherein the distal end of the shoulder portion inclines inwards the axis of the delivery pipe, so as to ensure that the manipulation member is clamped to the proximal end of the clamping member, so as to drive the processing units to rotate.

20. The multiple processing device for the endoscope of claim 18, wherein the clamping member comprises a pair of pins penetrating through the proximal end of the clamping arm, the pins comprises a first end and a second end, and the second end of the pin is a movable end, the first end of the pin is U-shaped via an integrally forming process, or the first end is provided with a limiting strip, which is configured to limit the pair of pins and fix the first end of the pin to the clamping arm.

21. The multiple processing device for the endoscope of claim 18, wherein the clamping member comprises a folded portion, which is defined by an arm surface of the clamping arm bending inwards, and the folded portion and the clamping arm are enclosed to define two end surfaces configured to abut against the concave portion.

22. The multiple processing device for the endoscope of claim 18, wherein each of the processing units further comprises a guiding member disposed between two clamping arms, when in the closed state, the manipulation member penetrates through the guiding member towards a direction of the head portion abutting against the clamping member under restriction of the guiding member.

23. The multiple processing device for the endoscope of claim 1, wherein a part of a sidewall of the proximal end of the clamping arm protruding outwards is defined as a locking portion, a part of a sidewall of the capsule bending inwards is defined as a clamping hole portion, and the clamping hole portion matches with the locking portion and is capable of limiting the clamping arm from moving towards a distal end of the capsule.

24. The multiple processing device for the endoscope of claim 1, wherein the delivery pipe comprises a first pipe near the distal end of the delivery pipe and a second end near the proximal end of the delivery pipe, and the first pipe is fixed to the second pipe,
wherein the first pipe is made by winding ribbon-shaped wires and the second pipe is made by winding round-shaped wires, and the first pipe is configured to accommodate the processing units.

25. A multiple processing device for an endoscope, comprising:
a processing component, wherein the processing component comprises at least two processing units, and a proximal end of processing units is provided with a first limiting portion that is capable of deforming or out of operation under an action of an external force;
a delivery component, wherein the delivery component comprises a delivery pipe accommodated in the processing component, a pushing pipe penetrating through the delivery pipe and an manipulation member penetrating through the pushing pipe, wherein a distal end of the pushing pipe abuts against a proximal processing unit of the processing units; and
an operating component, wherein the operating component comprises:
a handle portion combined with a proximal end of the delivery pipe, and
a slidable portion combined with a proximal end of the manipulation member, which is configured to control a distal processing unit of the of processing units to open and close; and
a pushing component disposed on the handle portion, wherein the pushing component comprises a fixing seat fixed to a proximal end of the delivery pipe;
wherein the handle portion is provided with a sliding groove for the fixing seat to slide, and the handle portion is provided with a marking portion configured for matching with the fixing seat and showing the number of the processing units.

26. The multiple processing device for the endoscope of claim 25, wherein the pushing component further comprises a locking member and a resetting member,
the locking member is connected to the fixing seat, the resetting member is disposed between the fixing seat and the locking member and abuts against the locking member, the locking member is provided with a first locking portion, the handle portion is provided with a second locking portion, and the first locking portion is capable of meshing with or separating from the second locking portion, so as to lock or unlock the locking member with the handle portion.

27. The multiple processing device for the endoscope of claim 25, wherein the handle portion is provided with a plurality of marking portions, which are disposed in equidistance along a moving direction of the fixing seat; and
the marking portions are figure notations, which are configured to show the number of remaining processing units.

28. The multiple processing device for the endoscope of claim 26, wherein the fixing seat is provided with a guiding column extending towards the locking member, the locking member is provided with a guiding groove through which the guiding column inserts, and the locking member is capable of moving along an axis of the guiding column relative to the locking member.

29. The multiple processing device for the endoscope of claim 26, wherein the locking member is further provided with an extension portion stretching out from a sidewall of the handle portion in a locked state, and the sidewall of the handle portion is provided with a sliding groove, wherein the extension portion is capable of stretching out from the sliding groove and sliding along the sliding groove.

30. The multiple processing device for the endoscope of claim 25, wherein the pushing component further comprises a crimper, which is sleeved on the pushing pipe and fixed to the fixing seat.

31. The multiple processing device for the endoscope of claim 30, wherein a number of the crimper is two, and the two crimpers are fixed to two ends of the fixing seat, respectively.

32. A method for use a multiple processing device for an endoscope, wherein the multiple processing device for the endoscope comprises a processing component, a delivery component and a pushing component,
wherein the processing component comprises at least two processing units;
the pushing component comprises a delivery pipe configured for accommodating the processing component, a pushing pipe penetrating through the delivery pipe, and a manipulation member penetrating through the pushing pipe, wherein a distal end of the pushing pipe abuts against a proximal processing unit, and the pushing component is fixed to a proximal end of the pushing pipe;
the method comprises the following steps:
releasing a distal processing unit in the delivery pipe, and driving the manipulation member to move towards a proximal end of the delivery pipe, until a first user feedback, which shows a first processing unit is combined with a distal end of the manipulation member, is obtained;
driving the pushing component to move towards a distal end of the delivery pipe, and driving the first processing unit to move towards a distal end of the delivery pipe via the pushing pipe fixed to the pushing component, until a second user feedback, which shows the first processing unit is combined with the distal end of the delivery pipe, is obtained; and
after the second user feedback is obtained, driving the distal processing component with the operating component to freely open or closed to obtain an expected state.

33. The method of claim 32, wherein the pushing component comprises a fixing seat, a locking member and a resetting member, wherein the resetting member is disposed between the fixing seat and the locking member and abuts against the fixing seat and the locking member, wherein the locking member is provided with a first locking portion matching with a second locking portion disposed on the handle portion;
the step of driving the pushing component to move towards a distal end of the delivery pipe, and driving the first processing unit to move towards a distal end of the delivery pipe via the pushing pipe fixed to the pushing component, until a second user feedback, which shows the first processing unit is combined with the distal end of the delivery pipe, is obtained further comprises:
when the pushing pipe is moved, pressing the locking member to make the first locking portion and the second locking portion be unlocked; and
after the second user feedback is obtained, releasing the locking member to make the first locking portion and the second locking portion be locked.
